# EUROPÄISCHE PATENTSCHRIFT

(11) **EP 2 311 860 B1**
(45) Veröffentlichungstag und Bekanntmachung des Hinweises auf die Patenterteilung: **07.09.2016**
(21) Anmeldenummer: 10010633.5
(22) Anmeldetag: 12.03.2003
(51) Int. Cl.: A61K 38/00, A61K 39/00, A61K 39/395, A61K 48/00, G01N 33/574, C07K 16/30, C07K 14/47, C12Q 1/68

(54) **Differentiell in Tumoren exprimierte Genprodukte und deren Verwendung**
Differential in tumour gene products and use of same
Produits géniques d'expression différentielle dans les tumeurs et leur utilisation

(30) Priorität: 13.03.2002 DE 10211088
(43) Veröffentlichungstag der Anmeldung: 20.04.2011
(62) Teilanmeldung aus: 03714811.1
(73) Patentinhaber: BioNTech AG, 55131 Mainz (DE)
(72) Erfinder: Sahin, Ugur, 55116 Mainz (DE); Türeci, Özlem, 55116 Mainz (DE); Koslowski, Michael, 85764 Oberschleißheim (DE)
(74) Vertreter: Schnappauf, Georg

(56) Entgegenhaltungen:
- WO-A2-01/51628
- WO-A2-01/60860
- SAHIN U ET AL: "Serological identification of human tumor antigens", CURRENT OPINION IN IMMUNOLOGY, ELSEVIER, OXFORD, GB, Bd. 9, Nr. 5, 1. Oktober 1997 (1997-10-01) , Seiten 709-716, XP004313590, ISSN: 0952-7915, DOI: DOI:10.1016/S0952-7915(97)80053-2
- SCHEURLE DANIELA ET AL: "Cancer gene discovery using Digital Differential Display", CANCER RESEARCH, AMERICAN ASSOCIATION FOR CANCER RESEARCH, US, Bd. 60, Nr. 15, 1. August 2000 (2000-08-01), Seiten 4037-4043, XP002256957, ISSN: 0008-5472
- LUCAS S ET AL: "IDENTIFICATION OF A NEW MAGE GENE WITH TUMOR-SPECIFIC EXPRESSION BY REPRESENTATIONAL DIFFERENCE ANALYSIS", CANCER RESEARCH, AMERICAN ASSOCIATION FOR CANCER RESEARCH, US, Bd. 58, Nr. 4, 15. Februar 1998 (1998-02-15), Seiten 743-752, XP001120296, ISSN: 0008-5472
- MARTELANGE V ET AL: "Identification on a human sarcoma of two new genes with tumor-specific expression", CANCER RESEARCH, AMERICAN ASSOCIATION FOR CANCER RESEARCH, US, Bd. 60, Nr. 14, 15. Juli 2000 (2000-07-15) , Seiten 3848-3855, XP002256958, ISSN: 0008-5472

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfunktionen interventionell verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekularer Strukturen in den Krebszellen. Werden solche Tumor-assoziierten Strukturen vom spezifischen Immunsystem des tumortragenden Wirtes erkannt, spricht man von tumor-assoziierten Antigenen. An der spezifischen Erkennung von tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC-(Major Histocompatibility complex = HistokompatibilitätsAntigene) Klassen II bzw. I präsentiert werden, während B-Lymphozyten zirkulierende Antikörpermoleküle produzieren, die direkt an unprozessierte Antigene binden. Die potentielle klinisch-therapeutische Bedeutung von tumor-assoziierten Antigenen ergibt sich aus der Tatsache, dass die Erkennung von Antigenen auf neoplastischen Zellen durch das Immunsystem zur Initiierung von cytotoxischen Effektormechanismen führt und bei Vorhandensein von T-Helferzellen die Elimination der Krebszellen bewirken kann (Pardoll, Nat. Med 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-Lymphozyten (CTL) (van der Bruggen et al., Science 254:1643-7, 1991) bzw. mit zirkulierenden Autoantikörpern (Sahin et al., Curr. Opin. Immunol, 9:709-16, 1997) als Sonden cDNA-Expressionbanken von Tumoren systematisch auf tumor-assoziierte Antigene zu screenen. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumor-spezifischem Lysemuster, oder zirkulierende Autoantikörper wurden genutzt, um die respektiven Antigene zu klonieren.
Durch diese Ansätze sind in den letzten Jahren eine Vielzahl von Antigenen in verschiedenen Neoplasien definiert worden. Von großem Interesse ist dabei die Klasse der Cancer/Testis-Antigene (CTA). CTA und sie kodierende Gene (Cancer/Testis-Gene oder CTG) sind durch ihr charakteristisches Expressionsmuster definiert [Tureci et al, Mol Med Today. 3:342-9, 1997]. Sie finden sich nicht in Normalgeweben bis auf Testis bzw. Keimzellen, werden jedoch in einer Reihe von humanen Malignomen exprimiert und zwar nicht tumortypspezifisch, sondern mit unterschiedlicher Häufigkeit in Tumorentitäten ganz unterschiedlicher Herkunft (Chen & Old, Cancer J. Sci. Am. 5:16-7, 1999). Auch Serumreaktivitäten gegen CTA finden sich nicht in gesunden Kontrollen, sondern lediglich in Tumorpatienten. Insbesondere aufgrund ihrer Gewebeverteilung ist diese Antigenklasse von besonderem Wert für immuntherapeutische Vorhaben und wird in derzeit laufenden klinischen Patientenstudien getestet (Marchand et al., Int. J. Cancer 80:219-30, 1999; Knuth et al., Cancer Chemother. Pharmacol. 46: S46-51, 2000).
Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumoren z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche Wirkung der bisher entdeckten und genutzten tumor-assoziierten Antigene ergeben. Entsprechend kann nicht ausgeschlossen werden, dass spontane Immunantworten evozierende Proteine die falschen Zielstrukturen sind.

Es war die Aufgabe der vorliegenden Erfindung Zielstrukturen für eine Diagnose und Therapie von Krebserkrankungen bereitzustellen.

Diese Aufgabe wird erfindungsgemäß durch den Gegenstand der Patentansprüche gelöst.

Erfindungsgemäß wurde eine Strategie für eine Identifizierung und Bereitstellung Tumorassoziiert exprimierter Antigene und der dafür kodierenden Nukleinsäuren verfolgt. Diese Strategie beruht auf der Tatsache, dass Testis- und damit Keimzell-spezifsche Gene, die normalerweise in adulten Geweben silent sind, in Tumorzellen ektop und unerlaubt reaktiviert werden. Durch Datamining wird zunächst eine möglichst komplette Liste aller bekannten Testis-spezifischen Gene aufgestellt und diese sodann durch Expressionsanalysen mittels spezifischer RT-PCR auf ihre aberrante Aktivierung in Tumoren evaluiert. Datamining ist ein bekanntes Verfahren zur Identifizierung von Tumor-assoziierten Genen. Bei den herkömmlichen Strategien werden allerdings in der Regel Transkriptome von Normalgewebebanken elektronisch von Tumorgewebsbanken subtrahiert unter der Annahme, dass die verbleibenden Gene Tumor-spezifisch sind (Schmitt et al., Nucleic Acids Res. 27:4251-60, 1999; Vasmatzis et al., Proc. Natl. Acad. Sci. U S A. 95:300-4, 1998. Scheurle et al., Cancer Res. 60:4037-43, 2000).
Das erfindungsgemäße Konzept, das sich als viel erfolgreicher erwiesen hat, beruht jedoch darauf, Datamining zur elektronischen Extraktion aller Testis-spezifischer Gene zu nutzen und diese sodann auf ektope Expression in Tumoren zu evaluieren.

Somit offenbart die Erfindung in einem Aspekt eine Strategie zur Identifizierung von differentiell in Tumoren exprimierten Genen. Diese kombiniert Datamining von öffentlichen Sequenzbanken ("*in silico*") mit darauffolgenden evaluierenden labor-experimentellen ("wet bench") Untersuchungen.

Eine kombinierte Strategie basierend auf zwei unterschiedlichen bioinformatischen Skripten ermöglichte erfindungsgemäß die Identifizierung neuer Mitglieder der Cancer/Testis (CT) Genklasse. Diese sind bisher als rein Testis-, Keimzell-, oder Spermien-spezifisch eingestuft worden. Die Erkenntnis, dass diese Gene aberrant in Tumorzellen aktiviert werden, erlaubt, ihnen eine substantiell neue Qualität mit funktionellen Implikationen zuzuordnen. Die Identifizierung dieser tumor-assoziierten Gene und der dadurch kodierten Genprodukte erfolgte unabhängig von einer immunogenen Wirkung.

Die erfindungsgemäß identifizierten Tumor-assoziierten Antigene weisen eine Aminosäuresequenz auf, die von einer Nukleinsäure kodiert wird, die aus der Gruppe ausgewählt ist, bestehend aus (a) einer Nukleinsäure, die eine Nukleinsäuresequenz gemäß SEQ ID NO: 39umfasst, (b) einer Nukleinsäure, die in Bezug auf die Nukleinsäure unter (a) degeneriert ist und (c) einer Nukleinsäure, die zu der Nukleinsäure unter (a) oder (b) eine Sequenzidentität von mindestens 90% aufweist.

Die vorliegende Erfindung betrifft allgemein die Verwendung von erfindungsgemäß identifizierten Tumor-assoziierten Antigenen oder von Teilen davon, von dafür kodierenden Nukleinsäuren oder von Nukleinsäuren, die gegen die kodierenden Nukleinsäuren gerichtet sind oder von Antikörpern, die gegen die erfindungsgemäß identifizierten Tumor-assoziierten Antigene oder Teile davon gerichtet sind, für die Diagnose. Diese Nutzung kann einzelne, aber auch Kombinationen von mehreren dieser Antigene, funktionalen Fragmente, Nukleinsäuren, Antikörper etc. betreffen, in einer Ausführungsform auch in Kombination mit anderen tumor-assoziierten Genen und Antigenen für eine Diagnose und Verlaufskontrolle. Bevorzugte Erkrankungen für eine Diagnose sind solche, bei denen eine selektive Expression oder abnormale Expression von einem oder mehreren der erfindungsgemäß identifizierten Tumor-assoziierten Antigenen vorliegt.

Vorliegend werden auch Nukleinsäuren und Genprodukte offenbart, die tumorzellassoziiert exprimiert werden und die durch verändertes Spleißen (Spleißvarianten) bekannter Gene bzw. durch veränderte Translation unter Nutzung alternativer offener Leserahmen entstehen. Für die Entstehung von Spleißvarianten können verschiedenste Mechanismen ursächlich sein, beispielsweise
- die Nutzung variabler Transkriptionsinitiationsstellen
- die Nutzung zusätzlicher Exons
- vollständiges oder unvollständiges Aus Spleißen von einzelnen oder mehreren Exons,
- über Mutation veränderte Spleißregulatorsequenzen (Deletion bzw. Schaffung neuer Donor/Acceptorsequenzen),
- die unvollständige Elimination von Intronsequenzen.

Das veränderte Spleißen eines Gens führt zu einer veränderten Transkriptsequenz (Spleißvariante). Wird eine Spleißvariante im Bereich ihrer veränderten Sequenz translatiert, resultiert ein verändertes Protein, welches sich von dem ursprünglichen in Struktur und Funktion deutlich unterscheiden kann. Bei tumorassoziierten Spleißvarianten können tumorassoziierte Transkripte und tumorassoziierte Proteine/Antigene entstehen. Diese können als molekulare Marker sowohl zum Nachweis von Tumorzellen als auch zum therapeutischen Targeting von Tumoren genutzt werden. Die Detektion von Tumorzellen z.B. im Blut, Serum, Knochenmark, Sputum, Bronchial-Lavage, Körpersekreten und Gewebsbiopsien kann erfindungsgemäß z.B. nach Extraktion von Nukleinsäuren durch PCR-Amplifikation mit Spleißvariantenspezifischen Oligonukleotiden erfolgen. Zum Nachweis eignen sich erfindungsgemäß alle Sequenz-abhängigen Detektionssysteme. Neben der PCR sind diese z.B. Genchip-/Microarraysysteme, Northem-Blot, RNAse protection assays (RDA) und andere. Allen Detektionssystemen ist gemeinsam, dass die Detektion auf einer spezifischen Hybridisierung mit mindestens einer Spleißvarianten-spezifischen Nukleinsäuresequenz basiert. Die Detektion von Tumorzellen kann jedoch auch erfindungsgemäß durch Antikörper erfolgen, die ein durch die Spleißvariante kodiertes spezifisches Epitop erkennen. Für die Herstellung der Antikörper können Peptide zur Immunisierung verwendet werden, die für diese Spleißvariante spezifisch sind. Für die Immunisierung eignen sich besonders die Aminosäuren, die deutliche Epitopunterschiede zu der Variante(n) des Genprodukts aufweisen, welche(s) bevorzugt in gesunden Zellen gebildet wird. Der Nachweis der Tumorzellen mit Antikörper kann dabei an einer vom Patienten isolierten Probe oder als Imaging mit intravenös applizierten Antikörpern erfolgen. Neben der diagnostischen Nutzbarkeit stellen Spleißvarianten, die neue oder veränderte Epitope aufweisen, attraktive Targets für die Immuntherapie dar. Die erfindungsgemäßen Epitope können zum Targeting von therapeutisch wirksamen monoklonalen Antikörpern oder T-Lymphozyten genutzt werden. Bei der passiven Immuntherapie werden hierbei Antikörper oder T-Lymphozyten adoptiv transferriert, die Spleißvarianten-spezifische Epitope erkennen. Die Generierung von Antikörpern kann wie bei anderen Antigenen auch unter Nutzung von Standardtechnologien (Immunisierung von Tieren, Panningstrategien zur Isolation von rekombinanten Antikörpern) unter Nutzung von Polypeptiden, die diese Epitope beinhalten, erfolgen. Alternativ können zur Immunisierung Nukleinsäuren genutzt werden, die für Oligo- oder Polypeptide kodieren, die diese Epitope beinhalten. Verschiedene Techniken zur in vitro oder in vivo Generierung von epitopspezifischen T-Lymphozyten sind bekannt und ausführlich beschrieben z.B. (Kessler JH, et al. 2001, Sahin et al., 1997) und basieren ebenfalls auf der Nutzung von Oligo-oder Polypeptide, die die Spleißvarianten-spezifischen Epitope beinhalten oder Nukleinsäuren, die für diese kodieren. Oligo-oder Polypeptiden, die die Spleißvariantenspezifischen Epitope beinhalten oder Nukleinsäuren, die für diese Polypeptide kodieren sind auch für die Nutzung als pharmazeutisch wirksame Substanzen bei der aktiven Immuntherapie (Vakzinierung, Vakzintherapie) verwendbar.

In einem Aspekt wird eine pharmazeutische Zusammensetzung offenbart, umfassend ein Mittel, das das erfindungsgemäß identifizierte Tumor-assoziierte Antigen erkennt und vorzugsweise selektiv für Zellen ist, die eine Expression oder abnormale Expression eines erfindungsgemäß identifizierten Tumor-assoziierten Antigens aufweisen. Das Mittel kann in bestimmten Ausführungsformen die Induktion des Zelltods, die Reduktion des Zellwachstums, die Schädigung der Zellmembran oder die Sekretion von Zytokinen bewirken und weist vorzugsweise eine tumorhemmende Aktivität auf. In einer Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet, insbesondere ein komplementaktivierter Antikörper, der selektiv an das Tumor-assoziierte Antigen bindet. In einer weiteren Ausführungsform umfasst das Mittel mehrere Mittel, die jeweils selektiv verschiedene Tumor-assoziierte Antigene erkennen, wobei mindestens eines der Tumor-assoziierten Antigene ein erfindungsgemäß identifiziertes Tumor-assoziiertes Antigen ist. Die Erkennung muss nicht direkt mit einer Hemmung von Aktivität oder Expression des Antigens einhergehen. In diesem Aspekt dient das selektiv auf Tumoren beschränkte Antigen vorzugsweise als Markierung zur Rekrutierung von Effektormechanismen an diesen spezifischen Ort. In einer bevorzugten Ausführungsform ist das Mittel ein cytotoxischer T-Lymphozyt, der das Antigen auf einem HLA-Molekül erkennt und die derartig markierte Zelle lysiert. In einer weiteren Ausführungsform ist das Mittel ein Antikörper, der selektiv an das Tumor-assoziierte Antigen bindet und somit natürliche oder artifizielle Effektormechanismen zu dieser Zelle rekrutiert. In einer weiteren Ausführungsform ist das Mittel ein T-Helfer-Lymphozyt, der Effektorfunktionen von anderen Zellen, die spezifisch dieses Antigen erkennen, stärkt.

In einem Aspekt wird eine pharmazeutische Zusammensetzung offenbart, umfassend ein Mittel, das die Expression oder Aktivität eines erfindungsgemäß identifizierten Tumor-assoziierten Antigens hemmt. In einer bevorzugten Ausfährungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet. In einer weiteren Ausführungsform umfasst das Mittel mehrere Mittel, die jeweils selektiv die Expression oder Aktivität verschiedener Tumor-assoziierter Antigene hemmen, wobei mindestens eines der Tumor-assoziierten Antigene ein erfindungsgemäß identifiziertes Tumor-assoziiertes Antigen ist.

Des weiteren wird eine pharmazeutische Zusammensetzung offenbart, die ein Mittel umfasst, das bei einer Verabreichung selektiv die Menge an Komplexen zwischen einem HLA-Molekül und einem Peptidepitop aus dem erfindungsgemäß identifizierten Tumor-assoziierten Antigen erhöht. Das Mittel umfasst in einer Ausführungsform einen oder mehrere Bestandteile, die aus der Gruppe ausgewählt sind, bestehend aus (i) dem Tumor-assoziierten Antigen oder einem Teil davon, (ii) einer Nukleinsäure, die für das Tumor-assoziierte Antigen oder einen Teil davon kodiert, (iii) einer Wirtszelle, die das Tumor-assoziierte Antigen oder einen Teil davon exprimiert, und (iv) isolierten Komplexen zwischen Peptidepitopen aus dem Tumor-assoziierten Antigen und einem MHC-Molekül. In einer Ausführungsform umfasst das Mittel mehrere Mittel, die jeweils selektiv die Menge an Komplexen zwischen MHC-Molekülen und Peptidepitopen verschiedener Tumor-assoziierter Antigene erhöhen, wobei mindestens eines der Tumor-assoziierten Antigene ein erfindungsgemäß identifiziertes Tumor-assoziiertes Antigen ist.

Des weiteren wird eine pharmazeutische Zusammensetzung offenbart, die einen oder mehrer Bestandteile umfasst, die aus der Gruppe ausgewählt sind, bestehend aus (i) einem erfindungsgemäß identifizierten Tumor-assoziierten Antigen oder einem Teil davon, (ii) einer Nukleinsäure, die für ein erfindungsgemäß identifiziertes Tumor-assoziiertes Antigen oder einen Teil davon kodiert, (iii) einem Antikörper, der an ein erfindungsgemäß identifiziertes Tumor-assoziiertes Antigen oder einen Teil davon bindet, (iv) einer Antisense-Nukleinsäure, die spezifisch mit einer Nukleinsäure, die für ein erfindungsgemäß identifiziertes Tumor-assoziiertes Antigen kodiert, hybridisiert, (v) einer Wirtszelle, die ein erfindungsgemäß identifiziertes Tumor-assoziiertes Antigen oder einen Teil davon exprimiert, und (vi) isolierten Komplexen zwischen einem erfindungsgemäß identifizierten Tumor-assoziierten Antigen oder einem Teil davon und einem HLA-Molekül.

Eine Nukleinsäure, die für ein erfndungsgemäß identifiziertes Tumor-assoziiertes Antigen oder einen Teil davon kodiert, kann in der pharmazeutische Zusammensetzung in einem Expressionsvektor vorliegen und funktionell mit einem Promotor verbunden sein.

Eine in der offenbarten pharmazeutischen Zusammensetzung enthaltene Wirtszelle kann das Tumor-assoziierte Antigen oder den Teil davon sekretieren, auf der Oberfläche exprimieren oder kann zusätzlich ein HLA-Molekül exprimieren, das an das Tumor-assoziierte Antigen oder den Teil davon bindet. In einer Ausführungsform exprimiert die Wirtszelle das HLA-Molekül endogen. In einer weiteren Ausführungsform exprimiert die Wirtszelle das HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

Ein in der offenbarten pharmazeutischen Zusammensetzung enthaltener Antikörper kann ein monoklonaler Antikörper sein. In weiteren Ausführungsformen ist der Antikörper ein chimärer oder humanisierter Antikörper, ein Fragment eines natürlichen Antikörpers, oder ein synthetischer Antikörper, die durch kombinatorische Techniken hergestellt werden können. Der Antikörper kann mit einem therapeutisch oder diagnostisch nützlichen Mittel gekoppelt sein.

Eine in der offenbarten pharmazeutischen Zusammensetzung enthaltene Antisense-Nukleinsäure kann eine Sequenz von 6-50, insbesondere 10-30, 15-30 und 20-30 zusammenhängenden Nukleotiden aus der Nukleinsäure, die für das erfindungsgemäß identifizierte Tumor-assoziierte Antigen kodiert, umfassen.

In weiteren Ausführungsformen bindet ein durch die offenbarte pharmazeutische Zusammensetzung entweder direkt oder durch die Expression einer Nukleinsäure bereitgestelltes Tumor-assoziiertes Antigen oder ein Teil davon an MHC-Moleküle auf der Oberfläche von Zellen, wobei die Bindung vorzugsweise eine cytolytische Reaktion hervorruft und/oder eine Cytokinausschüttung induziert.

Die offenbarte pharmazeutische Zusammensetzung kann einen pharmazeutisch verträglichen Träger und/oder ein Adjuvans umfassen. Das Adjuvans kann aus Saponin, GM-CSF, CpG-Nukleotiden, RNA, einem Zytokin oder einem Chemokin ausgewählt sein. Die offenbarte pharmazeutische Zusammensetzung wird vorzugsweise zur Behandlung einer Erkrankung eingesetzt, die sich durch die selektive Expression oder abnormale Expression eines Tumor-assoziierten Antigens auszeichnet. In einer bevorzugten Ausführungsform ist die Erkrankung Krebs.

Des weiteren wird vorliegend ein Verfahren zur Behandlung oder Diagnose einer Erkrankung offenbart, die sich durch die Expression oder abnormale Expression eines oder mehrerer Tumor-assoziierter Antigene auszeichnet. In einer Ausführungsform umfasst die Behandlung die Verabreichung der vorliegend offenbarten pharmazeutischen Zusammensetzung.

In einem Aspekt wird ein Verfahren zur Diagnose einer Erkrankung offenbart, die sich durch die Expression oder abnormale Expression eines erfindungsgemäß identifizierten Tumor-assoziierten Antigens auszeichnet. Das Verfahren umfasst den Nachweis (i) einer Nukleinsäure, die für das Tumor-assoziierte Antigen kodiert, oder eines Teils davon und/oder (ii) den Nachweis des Tumor-assoziierten Antigens oder eines Teils davon, und/oder (iii) den Nachweis eines Antikörpers gegen das Tumor-assoziierte Antigen oder einen Teil davon und/oder (iv) den Nachweis von cytotoxischen oder Helfer-T-Lymphozyten, die für das Tumor-assoziierte Antigen oder einen Teil davon spezifisch sind in einer aus einem Patienten isolierten biologischen Probe. In bestimmten Ausführungsformen umfasst der Nachweis (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an cytotoxische oder Helfer-T-Lymphozyten, die für das Tumor-assoziierte Antigen oder Teile davon spezifisch sind, bindet und (ii) den Nachweis der Komplexbildung zwischen dem Mittel und der Nukleinsäure oder dem Teil davon, dem Tumor-assoziierten Antigen oder dem Teil davon, dem Antikörper oder den cytotoxischen oder Helfer-T-Lymphozyten. In einer Ausführungsform zeichnet sich die Erkrankung durch die Expression oder abnormale Expression mehrerer verschiedener Tumor-assoziierter Antigene aus und der Nachweis umfasst einen Nachweis mehrerer Nukleinsäuren, die für die mehreren verschiedenen Tumor-assoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden oder den Nachweis mehrerer cytotoxischer oder Helfer-T-Lymphozyten, die für die mehreren verschiedenen Tumor-assoziierten Antigene spezifisch sind. In einer weiteren Ausführungsform wird die isolierte biologische Probe aus dem Patienten mit einer vergleichbaren normalen biologischen Probe verglichen.

In einem weiteren Aspekt wird vorliegend ein Verfahren zur Bestimmung der Regression, des Verlaufs oder des Ausbruchs einer Erkrankung offenbart, die sich durch die Expression oder abnormale Expression eines erfindungsgemäß identifizierten Tumor-assoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, die aus der Gruppe ausgewählt sind, bestehend aus (i) der Menge der Nukleinsäure, die für das Tumor-assoziierte Antigen kodiert, oder eines Teil davon, (ii) der Menge des Tumor-assoziierten Antigens oder eines Teils davon, (iii) der Menge an Antikörpern, die an das Tumor-assoziierte Antigen oder einen Teil davon binden, und (iv) der Menge an cytolytischen T-Zellen oder Helfer-T-Zellen, die für einen Komplex zwischen dem Tumor-assoziierten Antigen oder einem Teil davon und einem MHC-Molekül spezifisch sind. Vorzugsweise umfasst das Verfahren die Bestimmung des oder der Parameter zu einem ersten Zeitpunkt in einer ersten Probe und zu einem zweiten Zeitpunkt in einer weiteren Probe, wobei durch einen Vergleich der beiden Proben der Verlauf der Erkrankung ermittelt wird. In bestimmten Ausführungsformen zeichnet sich die Erkrankung durch die Expression oder abnormale Expression mehrerer verschiedener Tumor-assoziierter Antigene aus und die Überwachung umfasst eine Überwachung (i) der Menge mehrerer Nukleinsäuren, die für die mehreren verschiedenen Tumor-assoziierten Antigene kodieren, oder von Teilen davon und/oder (ii) der Menge der mehreren verschiedenen Tumor-assoziierten Antigene oder von Teilen davon und/oder (iii) der Menge mehrerer Antikörper, die an die mehreren verschiedenen Tumor-assoziierten Antigene oder an Teile davon binden, und/oder (iv) der Menge mehrerer cytolytischer T-Zellen oder Helfer-T-Zellen, die für Komplexe zwischen den mehreren verschiedenen Tumor-assoziierten Antigenen oder von Teilen davon und MHC-Molekülen spezifisch sind.

Ein Nachweis einer Nukleinsäure oder eines Teils davon oder eine Überwachung der Menge einer Nukleinsäure oder eines Teils davon kann erfindungsgemäß mit einer Polynukleotid-Sonde erfolgen, die spezifisch mit der Nukleinsäure oder dem Teil davon hybridisiert oder kann durch selektive Amplifikation der Nukleinsäure oder des Teils davon erfolgen. In einer Ausführungsform umfasst die Polynukleotid-Sonde eine Sequenz von 6-50, insbesondere 10-30, 15-30 und 20-30 zusammenhängenden Nukleotiden aus der Nukleinsäure.

In bestimmten Ausführungsformen liegt das nachzuweisende Tumor-assoziierte Antigen oder der Teil davon intrazellulär oder auf der Zelloberfläche vor. Ein Nachweis eines Tumor-assoziierten Antigens oder eines Teils davon oder eine Überwachung der Menge eines Tumor-assoziierten Antigens oder eines Teils davon kann erfindungsgemäß mit einem Antikörper erfolgen, der spezifisch an das Tumor-assoziierte Antigen oder den Teil davon bindet.

In weiteren Ausführungsformen liegt das nachzuweisende Tumor-assoziierte Antigen oder der Teil davon in einem Komplex mit einem MHC-Molekül, insbesondere einem HLA-Molekül vor.

Ein Nachweis eines Antikörpers oder die Überwachung der Menge an Antikörpern kann mit einem Protein oder Peptid erfolgen, das spezifisch an den Antikörper bindet.

Ein Nachweis von cytolytischen T-Zellen oder Helfer-T-Zellen oder die Überwachung der Menge an cytolytischen T-Zellen oder Helfer-T-Zellen, die für Komplexe zwischen einem Antigen oder einem Teil davon und MHC-Molekülen spezifisch sind, kann mit einer Zelle erfolgen, die den Komplex zwischen dem Antigen oder dem Teil davon und einem MHC-Molekül präsentiert.

Die für einen Nachweis oder für eine Überwachung verwendete Polynukleotid-Sonde, der Antikörper, das Protein oder Peptid oder die Zelle sind vorzugsweise nachweisbar markiert. In bestimmten Ausführungsformen ist der nachweisbare Marker ein radioaktiver Marker oder ein Enzymmarker. Der Nachweis von T-Lymphozyten kann zusätzlich erfolgen durch Nachweis ihrer Proliferation, ihrer Zytokinproduktion, sowie ihret cytotoxischen Aktivität, die ausgelöst wird durch die spezifische Stimulation mit dem Komplex aus MHC und tumor-assoziiertem Antigen oder Teilen davon. Der Nachweis von T-Lymphozyten kann ferner erfolgen durch ein rekombinantes MHC-Molekül oder auch ein Komplex aus mehreren MHC-Molekülen, die beladen sind mit dem jeweiligen immunogenen Fragment aus einem oder mehreren der tumorassoziierten Antigene und durch Kontaktierung des spezifischen T-Zell-Rezeptors die spezifischen T-Lymphozyten identifizieren können.

In einem weiteren Aspekt wird ein Verfahren zur Behandlung, Diagnose oder Überwachung einer Erkrankung offenbart, die sich durch die Expression oder abnormale Expression eines erfindungsgemäß identifizierten Tumor-assoziierten Antigens auszeichnet, umfassend die Verabreichung eines Antikörpers, der an das Tumor-assoziierte Antigen oder einen Teil davon bindet und mit einem therapeutischen oder diagnostischen Mittel gekoppelt ist. Der Antikörper kann ein monoklonaler Antikörper sein. In weiteren Ausführungsformen ist der Antikörper ein chimärer oder humanisierter Antikörper oder ein Fragment eines natürlichen Antikörpers.

Die Beschreibung offenbart auch ein Verfahren zur Behandlung eines Patienten mit einer Erkrankung, die sich durch die Expression oder abnormale Expression eines erfindungsgemäß identifizierten Tumor-assoziierten Antigens auszeichnet, umfassend (i) die Entfernung einer Probe mit immunreaktiver Zellen aus dem Patienten, (ii) die Kontaktierung der Probe mit einer Wirtszelle, die das Tumor-assoziierte Antigen oder einen Teil davon exprimiert, unter Bedingungen, die eine Produktion cytolytischer T-Zellen gegen das Tumor-assoziierte Antigen oder einen Teil davon begünstigen, und (iii) das Einbringen der cytolytischen T-Zellen in den Patienten in einer Menge, die geeignet ist, Zellen zu lysieren, die das Tumor-assoziierte Antigen oder einen Teil davon exprimieren. Die Offenbarung betrifft ebenfalls die Klonierung des T-Zell-Rezeptors von cytolytischen T-Zellen gegen das Tumor-assoziierte Antigen. Dieser kann in andere T-Zellen transferiert werden, die damit die erwünschte Spezifität erhalten und wie unter (iii) in den Patienten eingebracht werden können.

In einer Ausführungsform exprimiert die Wirtszelle ein HLA-Molekül endogen. In einer weiteren Ausführungsform exprimiert die Wirtszelle ein HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einem weiteren Aspekt betrifft die Offenbarung ein Verfahren zur Behandlung eines Patienten mit einer Erkrankung, die sich durch die Expression oder abnormale Expression eines Tumor-assoziierten Antigens auszeichnet, umfassend (i) die Identifizierung einer für ein erfindungsgemäß identifiziertes Tumor-assoziiertes Antigen kodierenden Nukleinsäure, die von Zellen exprimiert wird, die mit der Erkrankung assoziiert sind, (ii) die Transfektion einer Wirtszelle mit der Nukleinsäure oder einem Teil davon, (iii) die Kultivierung der transfizierten Wirtszelle für eine Expression der Nukleinsäure (dies ist bei Erreichen einer hohen Transfektionsrate nicht obligat), und (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen. Das Verfahren kann ferner die Identifizierung eines MHC-Moleküls, das das Tumor-assoziierte Antigen oder einen Teil davon präsentiert, umfassen, wobei die Wirtszelle das identifizierte MHC-Molekül exprimiert und das Tumor-assoziierte Antigen oder einen Teil davon präsentiert. Die Immunreaktion kann eine B-Zellen-Reaktion oder eine T-Zellen-Reaktion umfassen. Des weiteren kann eine T-Zellen-Reaktion die Produktion von cytolytischen T-Zellen und/oder Helfer-T-Zellen umfassen, die spezifisch für die Wirtszellen sind, die das Tumor-assoziierte Antigen oder einen Teil davon präsentieren oder spezifisch für Zellen des Patienten sind, die das Tumor-assoziierte Antigen oder einen Teil davon exprimieren.

Die Offenbarung betrifft auch ein Verfahren zur Behandlung einer Erkrankung, die sich durch die Expression oder abnormale Expression eines erfindungsgemäß identifizierten Tumor-assoziierten Antigens auszeichnet, umfassend (i) die Identifikation von Zellen aus dem Patienten, die abnormale Mengen des Tumor-assoziierten Antigens exprimieren, (ii) die Isolierung einer Probe der Zellen, (üi) die Kultivierung der Zellen und (iv) das Einbringen der Zellen in den Patienten in einer Menge, die geeignet ist, eine Immunreaktion gegen die Zellen auszulösen.

Vorzugsweise sind die verwendeten Wirtszellen nicht-proliferativ oder werden nicht-proliferativ gemacht. Eine Erkrankung, die sich durch die Expression oder abnormale Expression eines Tumor-assoziierten Antigens auszeichnet, ist insbesondere Krebs.

Des weiteren betrifft die vorliegende Erfindung eine Nukleinsäure, die aus der Gruppe ausgewählt ist, bestehend aus (a) einer Nukleinsäure, die eine Nukleinsäuresequenz gemäß SEQ ID NO: 39 umfasst, (b) einer Nukleinsäure, die in Bezug auf die Nukleinsäure unter (a) degeneriert ist und (c) einer Nukleinsäure, die zu der Nukleinsäure unter (a) oder (b) mindestens eine Sequenzidentität von 90% aufweist.

Die erfindungsgemäßen Nukleinsäuren funktionell mit einem anderen Gen vorzugsweise in einem Expressionsvektor verbunden werden, und somit die selektive Expression dieses Gens in entsprechenden Zellen gewährleisten.

Vorliegend wird auch ein rekombinantes Nukleinsäuremolekül offenbart, insbesondere ein DNA- oder RNA-Molekül, das eine erfindungsgemäße Nukleinsäure umfasst.

Vorliegend werden auch Wirtszellen, die eine erfindungsgemäße Nukleinsäure oder ein rekombinantes Nukleinsäuremolekül, das eine erfindungsgemäße Nukleinsäure umfasst, enthalten.

Die Wirtszelle kann ferner eine Nukleinsäure umfassen, die für ein HLA-Moleküt kodiert. In einer Ausführungsform exprimiert die Wirtszelle das HLA-Molekül endogen. In einer weiteren Ausführungsform exprimiert die Wirtszelle das HLA-Molekül und/oder die erfindungsgemäße Nukleinsäure oder einen Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einer weiteren Ausführungsform betrifft die Erfindung Oligonukleotide, die mit einer erfindungsgemäß identifizierten Nukleinsäure hybridisieren und als genetische Sonden oder als "Antisense"-Moleküle verwendet werden können. Nukleinsäuremeleküle in der Form von Oligonukleotid-Primern oder kompetenten Proben, die mit einer erfindungsgemäß identifizierten Nukleinsäure oder Teilen davon hybridisieren, können zum Auffinden von Nukleinsäuren verwendet werden, die zu der erfindungsgemäß identifizierten Nukleinsäure homolog sind. PCR-Amplifikation, Southern- und Northem-Hybridisierung können zum Auffinden homologer Nukleinsäuren eingesetzt werden. Die Hybridisierung kann unter niedrig-, besser unter mittel- und am besten unter hoch-stringenten Bedingungen erfolgen. Der Begriff stringente Bedingungen" betrifft erfindungsgemäß Bedingungen, die eine spezifische Hybridisierung zwischen Polynukleotiden erlauben.

In einem weiteren Aspekt betrifft die Erfindung ein Protein oder Polypeptid, das von einer Nukleinsäure kodiert wird, die aus der Gruppe ausgewählt ist, bestehend aus (a) einer Nukleinsäure, die eine Nukleinsäuresequenz gemäß SEQ ID NO: 39 umfassst, (b) einer Nukleinsäure, die in Bezug auf die Nukleinsäure unter (a) degeneriert ist und (c) einer Nukleinsäure, die zu der Nukleinsäure unter (a) oder (b) eine Sequenzidentität von mindestens 90% aufweist.

In einem weiteren Aspekt wird ein immunogenes Fragment eines erfindungsgemäß identifizierten Tumor-assoziierten Antigens offenbart. Das Fragment bindet vorzugsweise an einen menschlichen HLA-Rezeptor oder menschlichen Antikörper. Vorzugsweise umfasst ein erfindungsgemäßes Fragment eine Sequenz von mindestens 6, insbesondere mindestens 8, mindestens 10, mindestens 12, mindestens 15, mindestens 20, mindestens 30 oder mindestens 50 Aminosäuren.

In einem weiteren Aspekt betrifft die Erfindung ein Mittel, das an ein erfindungsgemäß identifiziertes Tumor-assoziiertes Antigen oder an einen Teil davon bindet. In einer bevorzugten Ausführungsform ist das Mittel ein Antikörper. In weiteren Ausführungsformen ist der Antikörper ein chimärer, ein humanisierter oder mit kombinatorischen Techniken hergestellte Antikörper oder ein Fragment eines Antikörpers. Des weiteren betrifft die Erfindung einen Antikörper, der selektiv an einen Komplex aus (i) einem erfindungsgemäß identifizierten Tumor-assoziierten Antigen oder einem Teil davon und (ii) einem MHC-Molekül bindet, an das das erfindungsgemäß identifizierte Tumor-assoziierte Antigen oder der Teil davon bindet, wobei der Antiköper nicht alleine an (i) oder (ii) bindet. Ein erfindungsgemäßer Antikörper kann ein monoklonaler Antikörper sein. In weiteren Ausführungsformen ist der Antikörper ein chimärer oder humanisierter Antikörper oder ein Fragment eines natürlichen Antikörpers.

Des weiteren betrifft die Erfindung ein Konjugat zwischen einem erfindungsgemäßen Mittel, das an ein erfindungsgemäß identifiziertes Tumor-assoziiertes Antigen oder an einen Teil davon bindet, oder einem erfindungsgemäßen Antikörper und einem therapeutischen oder diagnostischen Mittel. In einer Ausführungsform ist das therapeutische oder diagnostische Mittel ein Toxin.

In einem weiteren Aspekt wird ein Kit zum Nachweis der Expression oder abnormalen Expression eines erfindungsgemäß identifizierten Tumor-assoziierten Antigens offenbart, umfassend Mittel zum Nachweis (i) der Nukleinsäure, die für das Tumor-assoziierte Antigen kodiert, oder eines Teils davon, (ii) des Tumor-assoziierten Antigens oder eines Teils davon, (iii) von Antikörpern, die an das Tumor-assoziierte Antigen oder einen Teil davon binden, und/oder (iv) von T-Zellen, die für einen Komplex zwischen dem Tumor-assoziierten Antigen oder einem Teil davon und einem MHC-Molekül spezifisch sind. In einer Ausführungsform sind die Mittel zum Nachweis der Nukleinsäure oder des Teils davon Nukleinsäuremoleküle für die selektive Amplifikation der Nukleinsäure, die insbesondere eine Sequenz von 6-50, insbesondere 10-30, 15-30 und 20-30 zusammenhängenden Nukleotiden aus der Nukleinsäure umfassen.

### Detaillierte Beschreibung der Erfindung

Erfindungsgemäß wird ein Gen beschrieben, das in Tumorzellen selektiv exprimiert oder aberrant exprimiert werden und ein Tumor-assoziiertes Antigen darstellt.

Das erfindungsgemäß identifizierte Gen stellt eine bevorzugte Zielstruktur für therapeutische Ansätze dar. Konzeptionell können die therapeutischen Ansätze auf eine Hemmung der Aktivität des selektiv exprimierten tumor-assoziierten Genproduktes zielen. Dies ist dann sinnvoll, wenn die aberrante respektive selektive Expression funktionell von tumorpathogenetischer Bedeutung ist und ihre Unterbindung mit einer selektiven Schädigung der entsprechenden Zellen einhergeht. Andere therapeutische Konzepte betrachten tumorassoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.

Mit "Derivat" einer Nukleinsäure ist erfindungsgemäß gemeint, dass einzelne oder multiple Nukleotidsubstitution, -deletion und/oder -addition in der Nukleinsäure vorliegen. Weiterhin umfasst der Begriff "Derivat" auch eine chemische Derivatisierung einer Nukleinsäure an einer Nukleotidbase, am Zucker oder am Phosphat. Der Begriff "Derivat" umfasst auch Nukleinsäuren, die nicht in der Natur vorkommende Nukleotide und Nukleotidanaloga enthalten.

Eine Nukleinsäure ist erfindungsgemäß vorzugsweise Desoxyribonukleinsäure (DNA) oder Ribonukleinsäure (RNA). Nukleinsäuren umfassen erfindungsgemäß genomische DNA, cDNA, mRNA, rekombinant hergestellte und chemisch synthetisierte Moleküle. Eine Nukleinsäure kann erfindungsgemäß als einzelsträngiges oder doppelsträngiges und lineares oder kovalent kreisförmig geschlossenes Molekül vorliegen.

Die erfindungsgemäß beschriebenen Nukleinsäuren sind vorzugsweise isoliert. Der Begriff "isolierte Nukleinsäure" bedeutet erfindungsgemäß, dass die Nukleinsäure (i) *in vitro* amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplex eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989 oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5mM NaH₂PO₄ (pH7), 0,5% SDS, 2mM EDTA). SSC ist 0,15 M Natriumchlorid/ 0,15 M Natriumcitrat, pH 7. Nach der Hybridisierung wird die Membran, auf die die DNA übertragen wurde beispielsweise in 2 x SSC bei Raumtemperatur und sodann in 0,1 - 0,5 x SSC/ 0,1 x SDS bei Temperaturen bis 68°C gewaschen.

Komplementäre Nukleinsäuren weisen erfindungsgemäß mindestens 40%, insbesondere mindestens 50%, mindestens 60%, mindestens 70%, mindestens 80%, mindestens 90% und vorzugsweise mindestens 95%, mindestens 98 oder mindestens 99% Identität der Nukleotide auf.

Nukleinsäuren, die für Tumor-assoziierte Antigene kodieren, können erfindungsgemäß alleine oder in Kombination mit anderen Nukleinsäuren, insbesondere heterologen Nukleinsäuren, vorliegen. In bevorzugten Ausführungsformen liegt eine Nukleinsäure funktionell in Verbindung mit Expressionskontrollsequenzen oder regulatorischen Sequenzen vor, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz" umfasst erfindungsgemäß Promotoren, Enhancer und andere Kontrollelemente, die die Expression eines Gens steuern. In bestimmten erfindungsgemäßen Ausführungsformen sind die Expressionskontrollsequenzen regulierbar. Die genaue Struktur von regulatorischen Sequenzen kann speziesabhängig oder zelltypusabhängig variieren, umfasst jedoch im allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt. Regulatorische Sequenzen können auch Enhancer-Sequenzen oder stromaufwärts gelegene Aktivatorsequenzen umfassen.

Zum einen können also die hier dargestellten tumorassoziierten Antigene mit beliebigen Expressionskontrollsequenzen und Promotoren kombiniert werden. Zum anderen aber können erfindungsgemäß die Promotoren der hier dargestellten tumor-assoziierten Genprodukte mit beliebigen anderen Genen kombiniert werden. Dies erlaubt, die selektive Aktivität dieser Promotoren zu nutzen.

Des weiteren kann eine Nukleinsäure erfindungsgemäß in Verbindung mit einer anderen Nukleinsäure vorliegen, die für ein Polypeptid kodiert, das eine Sekretion des durch die Nukleinsäure kodierten Proteins oder Polypeptids aus einer Wirtszelle steuert. Auch kann eine Nukleinsäure erfindungsgemäß in Verbindung mit einer anderen Nukleinsäure vorliegen, die für ein Polypeptid kodiert, das eine Verankerung des kodierten Proteins oder Polypeptids auf der Zellmembran der Wirtszelle oder seine Kompartimentalisierung in bestimmte Organellen dieser Zelle herbeiführt.

In einer bevorzugten Ausführungsform ist ein rekombinantes DNA-Molekül erfindungsgemäß ein Vektor, gegebenenfalls mit einem Promotor, der die Expression einer Nukleinsäure, z.B. einer Nukleinsäure, die für eine erfindungsgemäßes Tumor-assoziiertes Antigen kodiert, steuert. Der Begriff "Vektor" wird dabei in seiner allgemeinsten Bedeutung verwendet und umfasst jegliche intermediären Vehikel für eine Nukleinsäure, die es z.B. ermöglichen die Nukleinsäure in prokaryotische und/oder in eukaryotische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA- oder RNA-Viren angepasst sein. Vektoren umfassen Plasmide, Phagemide oder Virusgenome.

Die Nukleinsäuren, die für ein erfindungsgemäß identifiziertes Tumor-assoziiertes Antigen kodieren, können für eine Transfektion von Wirtszellen eingesetzt werden. Mit Nukleinsäuren ist dabei sowohl rekombinante DNA wie auch RNA gemeint. Rekombinante RNA kann durch in-vitro-Transkription von einer DNA-Matritze hergestellt werden. Sie kann des weiteren vor Applikation durch stabilisierende Sequenzen, Capping und Poly-Adenylierung modifiziert werden. Der Begriff "Wirtszelle" betrifft erfindungsgemäß jede Zelle, die mit einer exogenen Nukleinsäure transformierbar oder transfizierbar ist. Der Begriff "Wirtszellen" umfasst erfindungsgemäß prokaryontische (z.B. *E. coli*) oder eukaryontische (z.B. dendritische Zellen, B-Zellen, CHO-Zellen, COS-Zellen, K562-Zellen, Hefezellen und Insektenzellen). Besonders bevorzugt sind Säugerzellen wie Zellen aus Mensch, Maus, Hamster, Schwein, Ziege, Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, Monozyt oder ein Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird erfindungsgemäß in seiner allgemeinsten Bedeutung verwendet und umfasst die Produktion von RNA oder von RNA und Protein. Er umfasst auch eine teilweise Expression von Nukleinsäuren. Des weiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht) und die Enhancer-Promotor-Sequenzen von Cytomegalovirus (CMV).

In den Fällen der Erfindung, in denen ein HLA-Molekül ein Tumor-assoziiertes Antigen oder einen Teil davon präsentiert, kann ein Expressionsvektor auch eine Nukleinsäuresequenz umfassen, das für das HLA-Molekül kodiert. Die Nukleinsäuresequenz, die für das HLA-Molekül kodiert, kann auf demselben Expressionsvektor wie die Nukleinsäure, die für das Tumor-assoziierte Antigen oder den Teil davon kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Tumor-assoziierte Antigen oder den Teil davon noch das HLA-Molekül exprimiert, werden beide dafür kodierenden Nukleinsäuren entweder auf demselben Expressionsvektor oder auf verschiedenen Expressionsvektoren in die Zelle transfiziert. Falls die Zelle bereits das HLA-Molekül exprimiert, kann nur die Nukleinsäuresequenz, die für das Tumor-assoziierte Antigen oder den Teil davon kodiert, in die Zelle transfiziert werden.

Erfindungsgemäß umfasst sind Kits zur Amplifikation einer Nukleinsäure, die für ein Tumor-assoziiertes Antigen kodiert. Solche Kits umfassen beispielsweise ein Paar von Amplifikationsprimern, die an die Nukleinsäure hybridisieren, die für das Tumor-assoziierte Antigen kodiert. Die Primer umfassen vorzugsweise eine Sequenz von 6-50, insbesondere 10-30, 15-30 und 20-30 zusammenhängenden Nukleotiden aus der Nukleinsäure und sind nicht-überlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt.

"Antisense"-Moleküle oder "Antisense"-Nukleinsäuren können zur Regulierung, insbesondere der Reduktion der Expression einer Nukleinsäure verwendet werden. Der Begriff "Antisense-Molekül" oder "Antisense-Nukleinsäure" betrifft erfindungsgemäß ein Oligonukleotid, das ein Oligoribonukleotid, Oligodesoxyribonukleotid, modifiziertes Oligoribonukleotid oder modifiziertes Oligodesoxyribonukleotid ist und das unter physiologischen Bedingungen an DNA, die ein bestimmtes Gen umfasst, oder mRNA dieses Gens hybridisiert, wodurch die Transkription dieses Gens und/oder die Translation dieser mRNA gehemmt wird. Ein "Antisense-Molekül" umfasst erfindungsgemäß auch ein Konstrukt, das eine Nukleinsäure oder einen Teil davon in reverser Orientierung in Bezug auf ihren natürlichen Promotor enthält. Ein Antisense-Transkript einer Nukleinsäure oder eines Teils davon kann eine Duplex mit der natürlich vorkommenden mRNA, die das Enzym spezifiziert, eingehen und so eine Akkumulation von oder die Translation der mRNA in das aktive Enzym verhindern. Eine weitere Möglichkeit ist die Verwendung von Ribozymen zur Inaktivierung einer Nukleinsäure. Bevorzugte erfindungsgemäße Antisense-Oligonukleotide weisen eine Sequenz von 6-50, insbesondere 10-30, 15-30 und 20-30 zusammenhängenden Nukleotiden aus der Ziel-Nukleinsäure auf und sind vorzugsweise vollständig zu der Ziel-Nukleinsäure oder einem Teil davon komplementär.

In bevorzugten Ausführungsformen hybridisiert das Antisense-Oligonukleotid mit einer N-terminalen oder 5'-stromaufwärts gelegenen Stelle wie einer Translationsinitiations-, Transkriptionsinitiations- oder Promotorstelle. In weiteren Ausführungsformen hybridisiert das Antisense-Oligonukleotid mit einer 3'-nicht-translatierten Region oder mRNA-Splicing-Stelle.

In einer Ausführungsform besteht ein erfindungsgemäßes Oligonukleotid aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

In bevorzugten Ausführungsformen ist ein erfindungsgemäßes Oligonukleotid ein "modifiziertes" Oligonukleotid. Dabei kann das Oligonukleotid, um beispielsweise seine Stabilität oder therapeutische Wirksamkeit zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet erfindungsgemäß ein Oligonukleotid bei dem (i) mindestens zwei seiner Nukleotide durch eine synthetische Internukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die erfindungsgemäß beschriebenen Proteine und Polypeptide sind vorzugsweise isoliert. Die Begriffe "isoliertes Protein" oder "isoliertes Polypeptid" bedeuten, dass das Protein oder Polypeptid von seiner natürlichen Umgebung getrennt ist. Ein isoliertes Protein oder Polypeptid kann in einem im wesentlichen aufgereinigten Zustand vorliegen. Der Begriff "im wesentlichen aufgereinigt" bedeutet, dass das Protein oder Polypeptid im wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay oder als Therapeutika einsetzbar. Erfindungsgemäß beschriebene Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften ersetzt, wie Hydrophobizität, Hydrophilizität, Elektronegativität, Volumen der Seitenkette und ähnliches (konservative Substitution). Konservative Substitutionen betreffen beispielsweise den Austausch einer Aminosäure durch eine andere, nachstehend in derselben Gruppe wie die substituierte Aminosäure aufgeführte Aminosäure:
1. kleine aliphatische, nicht-polare oder leicht-polare Reste: Ala, Ser, Thr (Pro, Gly)
2. negativ geladene Reste und ihre Amide: Asn, Asp, Glu, Gin
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. al. (1989) ausführlich beschrieben.

"Derivate" von Proteinen oder Polypeptiden umfassen erfindungsgemäß auch einzelne oder multiple Substitutionen, Deletionen und/oder Additionen jeglicher Moleküle, die mit dem Enzym assoziiert sind, wie Kohlenhydrate, Lipide und/oder Proteine oder Polypeptide. Ferner erstreckt sich der Begriff "Derivat" auch auf alle funktionellen chemischen Äquivalente der Proteine oder Polypeptide.

Ein Teil oder Fragment eines Tumor-assoziierten Antigens weist erfindungsgemäß eine funktionelle Eigenschaft des Polypeptids auf, aus dem es abgeleitet sind. Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität. Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer T-Zellen beruhen. Vorzugsweise umfasst ein erfindungsgemäßer Teil oder Fragment eines Tumor-assoziierten Antigens eine Sequenz von mindestens 6, insbesondere mindestens 8, mindestens 10, mindestens 12, mindestens 15, mindestens 20, mindestens 30 oder mindestens 50 aufeinanderfolgenden Aminosäuren aus dem Tumor-assoziierten Antigen.

Ein Teil oder ein Fragment einer Nukleinsäure, die für ein Tumor-assoziiertes Antigen kodiert, betrifft erfindungsgemäß den Teil der Nukleinsäure, der zumindest für das Tumor-assoziierte Antigen kodiert und/oder für einen Teil oder ein Fragment des Tumor-assoziierten Antigens wie vorstehend definiert kodiert.

Die Isolierung und Identifizierung von Genen, die für Tumor-assoziierte Antigene kodieren, ermöglicht auch die Diagnose einer Erkrankung, die sich durch die Expression von einem oder mehreren Tumor-assoziierten Antigenen auszeichnet. Diese Verfahren umfassen die Bestimmung einer oder mehrerer Nukleinsäuren, die für ein Tumor-assoziiertes Antigen kodieren, und/oder die Bestimmung der kodierten Tumor-assoziierten Antigene und/oder von davon abgeleiteten Peptiden. Eine Bestimmung der Nukleinsäure kann in herkömmlicher Weise erfolgen, einschließlich durch Polymerase-Kettenreaktion oder Hybridisierung mit einer markierten Sonde. Eine Bestimmung von Tumor-assoziierten Antigenen oder davon abgeleiteten Peptiden kann durch ein Screening von Patienten-Antiseren in Bezug auf eine Erkennung des Antigens und/oder der Peptide erfolgen. Sie kann auch erfolgen durch ein Screening von T-Zellen des Patienten auf Spezifität für das entsprechende tumor-assoziierte Antigen.

Die vorliegende Erfindung ermöglicht auch die Isolierung von Proteinen, die an hier beschriebene Tumor-assoziierte Antigene binden, einschließlich Antikörper und zelluläre Bindepartner der Tumor-assoziierten Antigene.

Vorliegend werden auch in bestimmten Ausführungsformen "dominant negative" Polypeptide offenbart, die von Tumor-assoziierten Antigenen abgeleitet sind. Ein dominant negatives Polypeptid ist eine inaktive Variante eines Proteins, die durch Interaktion mit der zellulären Maschinerie ein aktives Protein von seiner Interaktion mit der zellulären Maschinerie verdrängt oder mit dem aktiven Protein kompetitiert, wodurch die Wirkung des aktiven Proteins verringert wird. Zum Beispiel kann ein dominant negativer Rezeptor, der einen Liganden bindet, jedoch kein Signal in Reaktion auf die Bindung des Liganden erzeugt, die biologische Wirkung des Liganden verringern. In ähnlicher Weise kann eine dominant negative katalytisch-inaktive Kinase, die normalerweise mit Zielproteinen interagiert, jedoch die Zielproteine nicht phosphoryliert, die Phosphorylierung der Zielproteine in Reaktion auf ein zelluläres Signal verringern. In ähnlicher Weise kann ein dominant negativer Transkriptionsfaktor, der an eine Promotorstelle in der Kontrollregion eines Gens bindet, jedoch die Transkription des Gens nicht erhöht, die Wirkung eines normalen Transkriptionsfaktors durch die Besetzung von Promotorbindestellen ohne eine Erhöhung der Transkription verringern.

Das Ergebnis der Expression eines dominant negativen Polypeptids in einer Zelle ist eine Verringerung der Funktion aktiver Proteine. Der Fachmann kann dominant negative Varianten eines Proteins beispielsweise durch herkömmliche Mutageneseverfahren und Bewerten der dominant negativen Wirkung des Varianten-Polypeptids herstellen.

Denkbar sind auch Stoffe wie Polypeptide, die an Tumor-assoziierte Antigene binden. Solche Bindestoffe können z.B. in Screening-Assays für einen Nachweis von Tumor-assoziierten Antigenen und Komplexen von Tumor-assoziierten Antigenen mit ihren Bindepartnern sowie bei einer Aufreinigung der Tumor-assoziierten Antigene und von Komplexen davon mit ihren Bindepartnern Verwendung finden. Solche Stoffe können auch für eine Hemmung der Aktivität Tumor-assoziierter Antigene beispielsweise durch Bindung an solche Antigene Verwendung finden.

Erfindungsgemäß umfasst sind auch Bindestoffe wie z.B. Antikörper oder Antikörperfragmente, die die Fähigkeit aufweisen, selektiv an Tumor-assoziierte Antigene zu binden. Antikörper umfassen polyklonale und monoklonale Antikörper, die in herkömmlicher Weise hergestellt werden.

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Zum Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörper bezeichnet.

Erfindungsgemäß werden auch F(ab')₂-, Fab-, Fv- und Fd-Fragmente von Antikörpern, chimäre Antikörper, bei denen die Fc- und/oder FR und/oder CDR1- und/oder CDR2-und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden, bereitgestellt. Erfindungsgemäß umfasst sind auch sogenannte einzelkettige Antikörper.

Denkbar sind auch Polypeptide, die spezifisch an Tumor-assoziierte Antigene binden. Beispielsweise können solche Polypeptid-Bindestoffe durch degenerierte Peptid-Bibliotheken bereitgestellt werden, die einfach in Lösung in einer immobilisierten Form oder als Phagen-Display-Bibliotheken hergestellt werden können. Kombinatorische Bibliotheken aus Peptiden mit einer oder mehreren Aminosäuren können ebenfalls hergestellt werden. Ferner können Bibliotheken aus Peptoiden und nicht-peptidischen synthetischen Resten hergestellt werden.

Phagen-Display kann besonders wirksam bei der Identifizierung erfindungsgemäßer Bindepeptide sein. Dabei wird beispielsweise eine Phagen-Bibliothek (durch Verwendung beispielsweise des m13-, fd- oder lambda-Phagen) hergestellt, die Inserts einer Länge von 4 bis etwa 80 Aminosäureresten präsentiert. Es werden sodann Phagen ausgewählt, die Inserts tragen, die an das Tumor-assoziierte Antigen binden. Dieser Prozess kann über mehrere Zyklen einer Rückselektion von Phagen wiederholt werden, die an das Tumor-assoziierte Antigen binden. Wiederholte Runden führen zu einer Anreicherung von Phagen, die bestimmte Sequenzen tragen. Es kann eine Analyse von DNA-Sequenzen erfolgen, um die Sequenzen der exprimierten Polypeptide zu identifizieren. Der kleinste lineare Anteil der Sequenz, der an das Tumor-assoziierte Antigen bindet, kann bestimmt werden. Das "twohybrid-System" aus Hefe kann auch für die Identifizierung von Polypeptiden eingesetzt werden, die an ein Tumor-assoziiertes Antigen binden. Erfindungsgemäß beschriebene Tumor-assoziierte Antigene oder Fragmente davon können für ein Screening von Peptid-Bibliotheken, einschließlich Phagen-Display-Bibliotheken, eingesetzt werden, um Peptid-Bindepartner der Tumor-assoziierten Antigene zu identifizieren und selektieren. Solche Moleküle können beispielsweise für Screening-Assays, Aufreinigungsprotokolle, für eine Interferenz mit der Funktion des Tumor-assoziierten Antigens und für andere Zwecke, die dem Fachmann bekannt sind, verwendet werden.

Die vorstehend beschriebenen Antikörper und andere Bindemoleküle können beispielsweise für die Identifizierung von Gewebe verwendet werden, das ein Tumor-assoziiertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, Iocetaminsäure, lopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluorin-18 und Carbon-11, gamma-Emitter wie Iodin-123, Technitium-99m, Iod-131 und Indium-111, Nuklide für magnetische Kernresonanz wie Fluorin und Gadolinium. Der Begriff "therapeutisch nützlicher Stoff' meint erfindungsgemäß jedes therapeutische Molekül, das wunschgemäß selektiv zu einer Zelle gerührt wird, die ein oder mehrere Tumor-assoziierte Antigene exprimiert, einschließlich Antikrebsmittel, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabidin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner) beschrieben. Toxine können Proteine wie Pokeweed-antivirales Protein, Choleratoxin, Pertussistoxin, Ricin, Gelonin, Abrin, Diphtherie-Exotoxin oder *Pseudomonas-*Exotoxin sein. Toxinreste können auch Hochenergie-emittierende Radionuklide wie Kobalt-60 sein.

Der Begriff "Patient" bedeutet erfindungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausführungsform ist der Patient ein Mensch.

Der Begriff "Erkrankung" betrifft erfindungsgemäß jeden pathologischen Zustand, bei dem Tumor-assoziierte Antigene exprimiert oder abnormal exprimiert werden. "Abnormale Expression" bedeutet erfindungsgemäß, dass die Expression gegenüber dem Zustand bei einem gesunden Individuum verändert, vorzugsweise erhöht ist. Eine Erhöhung der Expression betrifft eine Erhöhung um mindestens 10%, insbesondere mindestens 20%, mindestens 50% oder mindestens 100%. In einer Ausführungsform wird das Tumor-assoziierte Antigen nur in Gewebe eines erkrankten Individuums exprimiert, während die Expression bei einem gesunden Individuum reprimiert ist. Ein Beispiel einer solchen Erkrankung ist Krebs, insbesondere Seminome, Melanome, Teratome, Gliome, Kolorektal-, Brust-, Prostata-, Gebärmutter-, Ovarial-, und Lungenkrebs.

Eine biologische Probe kann erfindungsgemäß eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Urin, Fäces oder anderen Körperflüssigkeiten.

Der Begriff "immunreaktive Zelle" bedeutet erfindungsgemäß eine Zelle, die in eine Immunzelle (wie B-Zelle, T-Helferzelle oder cytolytische T-Zelle) bei geeigneter Stimulierung reifen kann. Immunreaktive Zellen umfassen CD34⁺ hämatopoietische Stammzellen, unreife und reife T-Zellen sowie unreife und reife B-Zellen. Falls die Herstellung cytolytischer oder Helfer T-Zellen, die ein Tumor-assoziiertes Antigen erkennen, gewünscht ist, wird die immunreaktive Zelle mit einer Zelle, die ein Tumor-assoziiertes Antigen exprimiert, unter Bedingungen in Kontakt gebracht, die eine Produktion, Differenzierung und/oder Selektion von cytolytischen sowie Helfer T-Zellen begünstigen. Die Differenzierung von T-Zell-Vorläufern in eine cytolytische T-Zelle bei einer Exposition gegenüber einem Antigen ist ähnlich zur klonalen Selektion des Immunsystems.

Manche therapeutische Verfahren beruhen auf einer Reaktion des Immunsystems eines Patienten, die zu einer Lyse Antigen-präsentierender Zellen führt, wie Krebszellen, die ein oder mehrere Tumor-assoziierte Antigene präsentieren. Dabei werden beispielsweise autologe cytotoxische T-Lymphozyten, die für einen Komplex aus einem Tumor-assoziierten Antigen und einem MHC-Molekül spezifisch sind, an einen Patienten mit einer Zellabnormalie verabreicht Die Produktion solcher cytotoxischer T-Lymphozyten *in vitro* ist bekannt Ein Beispiel für ein Verfahren zur Differenzierung von T-Zellen findet sich in der WO-A-9633265. Im Allgemeinen wird eine Probe mit Zellen wie Blutzellen aus dem Patienten entnommen und die Zellen werden mit einer Zelle in Kontakt gebracht, die den Komplex präsentiert und eine Vermehrung von cytotoxischen T-Lymphozyten auslösen kann (z.B. dendritische Zellen). Die Zielzelle kann eine transfizierte Zelle wie eine COS-Zelle sein. Diese transfizierten Zellen präsentieren den gewünschten Komplex auf ihrer Oberfläche und stimulieren bei einer Kontaktierung mit cytotoxischen T-Lymphozyten deren Vermehrung. Die klonal expandierten autologen cytotoxischen T-Lymphozyten werden sodann an den Patienten verabreicht.

Bei einem anderen Verfahren zur Selektion Antigen-spezifischer cytotoxischer T-Lymphozyten werden fluorogene Tetramere von MHC-Klasse I-Molekül/Peptid-Komplexen für einen Nachweis spezifischer Klone von cytotoxischen T-Lymphozyten verwendet (Altman et al., Science 274:94-96, 1996; Dunbar et al., Curr. Biol. 8:413-416, 1998). Lösliche MHC-Klasse I-Moleküle werden *in vitro* in Gegenwart von β₂-Mikroglobulin und eines Peptid-Antigens, das an das Klasse I-Molekül bindet, gefaltet. Nach Aufreinigung der MHC/Peptid-Komplexe werden diese mit Biotin markiert. Tetramere werden durch Mischen der biotinylierten Peptid-MHC-Komplexe mit markiertem Avidin (z.B. Phycoerythrin) bei einem molaren Verhältnis von 4:1 gebildet. Tetramere werden sodann mit cytotoxischen T-Lymphozyten wie peripherem Blut oder Lymphknoten in Kontakt gebracht. Die Tetramere binden an cytotoxische T-Lymphozyten, die den Peptid-Antigen/MHC-Klasse I-Komplex erkennen. Zellen, die an die Tetramere gebunden werden, können durch Fluoreszenzgesteuerte Zellsortierung für eine Isolierung reaktiver cytotoxischer T-Lymphozyten sortiert werden. Die isolierten cytotoxischen T-Lymphozyten können sodann *in vitro* vermehrt werden.

Bei einem therapeutischen Verfahren, das als adoptiver Transfer bezeichnet wird (Greenberg, J. Immunol. 136(5):1917, 1986; Riddel et al., Science 257:238, 1992; Lynch et al., Eur. J. Immunol. 21:1403-1410, 1991; Kast et al., Cell 59:603-614, 1989), werden Zellen, die den gewünschten Komplex präsentieren (z.B. dendritische Zellen) mit cytotoxischen T-Lymphozyten des zu behandelnden Patienten kombiniert, was zu einer Vermehrung spezifischer cytotoxischer T-Lymphozyten führt. Die vermehrten cytotoxischen T-Lymphozyten werden sodann an einen Patienten mit einer zellulären Abnormalie verabreicht, die sich durch bestimmte abnormale Zellen auszeichnet, die den spezifischen Komplex präsentieren. Die cytotoxischen T-Lymphozyten lysieren sodann die abnormalen Zellen, wodurch eine gewünschte therapeutische Wirkung erreicht wird.
Oft lassen sich aus dem T-Zell-Repertoire eines Patienten lediglich niodrig-affine T-Zellen gegen einen solchen spezifischen Komplex vermehren, da die hochaffinen durch Toleranzentwicklung ausgelöscht worden sind. Eine Alternative kann hier ein Transfer des T-Zell-Rezeptors selbst sein. Hierfür werden ebenfalls Zellen, die den gewünschten Komplex präsentieren (z.B. dendritische Zellen) mit cytotoxischen T-Lymphozyten von Gesunden kombiniert. Dies führt zu einer Vermehrung hochaffiner spezifischer cytotoxischer T-Lymphozyten, wenn der Spender mit dem spezifischen Komplex bisher keinen Kontakt hatte. Der hochaffine T-Zell-Rezeptor aus diesen vermehrten spezifischen T-Lymphozyten wird kloniert und kann durch Gentransfer z.B. mit retroviralen Vektoren beliebig in T-Zellen von anderen Patienten transduziert werden Adoptiver Transfer erfolgt dann mit diesen genetisch veränderten T-Lymphozyten (Stanislawski et al., Nat Immunol. 2:962-70, 2001 ; Kessels et al., Nat Immunol. 2:957-61, 2001).

Die vorstehenden therapeutischen Aspekte gehen davon aus, dass zumindest manche der abnormalen Zellen des Patienten einen Komplex aus einem Tumor-assoziierten Antigen und einem HLA-Molekül präsentieren. Eine Identifizierung solcher Zellen kann in an sich bekannter Weise erfolgen. Sobald Zellen, die den Komplex präsentieren, identifiziert wurden, können sie mit einer Probe aus dem Patienten, die cytotoxische T-Lymphozyten enthält, kombiniert werden. Falls die Zellen, die den Komplex präsentieren, durch die cytotoxischen T-Lymphozyten lysiert werden, kann angenommen werden, dass ein Tumor-assoziiertes Antigen präsentiert wird.

Der adoptive Transfer ist nicht die einzige Therapieform, die erfindungsgemäß anwendbar ist. Cytotoxische T-Lymphozyten können auch *in vivo* in an sich bekannter Weise erzeugt werden. Bei einem Verfahren werden nicht-proliferative Zellen verwendet, die den Komplex exprimieren. Die Zellen, die dabei verwendet werden, werden diejenigen sein, die normalerweise den Komplex exprimieren, wie bestrahlte Tumorzellen oder Zellen, die mit einem oder beiden Genen transfiziert wurden, die für eine Präsentation des Komplexes notwendig sind (d.h. das Antigene Peptid und das präsentierende HLA-Molekül). Verschiedene Zelltypen können eingesetzt werden. Des weiteren können Vektoren verwendet werden, die eines oder beide der interessierenden Gene tragen. Virale oder bakterielle Vektoren sind besonders bevorzugt. Zum Beispiel können Nukleinsäuren, die für ein Tumor-assoziiertes Antigen oder einen Teil davon kodieren, funktionell mit Promotor- und Enhancersequenzen verknüpft werden, die eine Expression des Tumor-assoziierten Antigens oder eines Fragments davon in bestimmten Geweben oder Zelltypen steuern. Die Nukleinsäure kann in einen Expressionsvektor eingebaut werden. Expressionsvektoren können nicht-modifizierte extrachromosomale Nukleinsäuren, Plasmide oder virale Genome sein, in die eine Insertion exogener Nukleinsäuren möglich ist. Nukleinsäuren, die für ein Tumor-assoziiertes Antigen kodieren, können auch in ein retrovirales Genom inseriert werden, wodurch die Integration der Nukleinsäure in das Genom des Zielgewebes oder der Zielzelle ermöglicht wird. Bei diesen Systemen trägt ein Mikroorganismus wie Vacciniavirus, Poxvirus, Herpes simplex-Virus, Retrovirus oder Adenovirus das interessierende Gen und "infiziert" de facto Wirtszellen. Eine weitere bevorzugte Form ist die Einbringung des tumor-assoziierten Antigenes in Form von rekombinanter RNA. Diese kann z.B. durch liposomalen Transfer oder durch Elektroporation in Zellen eingebracht werden. Die resultierenden Zellen präsentieren den interessierenden Komplex und werden von autologen cytotoxischen T-Lymphozyten erkannt, die sich sodann vermehren.

Eine ähnliche Wirkung kann durch Kombination des Tumor-assoziierten Antigens oder eines Fragments davon mit einem Adjuvans erreicht werden, um einen Einbau in Antigen-präsentierende Zellen *in vivo* zu ermöglichen. Das tumor-assoziierte Antigen oder ein Fragment davon können als Protein, als DNA (z.B. innerhalb eines Vektors) oder als RNA repräsentiert sein. Das Tumor-assoziierte Antigen wird prozessiert, um einen Peptidpartner für das HLA-Molekül zu ergeben, während ein Fragment davon präsentiert werden kann, ohne dass eine weitere Prozessierung erforderlich ist. Letzteres ist insbesondere der Fall, wenn diese and HLA-Moleküle binden können. Verabreichungsformen, bei denen das Gesamt-Antigen *in vivo* von einer Dendritischen Zelle prozessiert wird, sind bevorzugt, da hier auch Helfer T-Zell-Antworten entstehen können. Eine effektive Immunantwort benötigt diese (Ossendorp et al, Immunol Lett. 74:75-9, 2000; Ossendorp et al, J. Exp. Med. 187:693-702, 1998). Im allgemeinen kann eine wirksame Menge des Tumor-assoziierten Antigens an einen Patienten z.B. durch eine intradermale Injektion verabreicht werden. Die Injektion kann aber auch intranodal in einen Lymphknoten erfolgen (Maloy et al, Proc Natl Acad Sci USA 98:3299-303, 2001). Sie kann auch in Kombination mit Reagenzien erfolgen, die eine Aufnahme in Dendritische Zellen erleichtern. Eine in vivo Bevorzugte Tumor-assoziierte Antigene umfassen diejenigen, die mit allogenen Krebs-Antiseren oder mit T-Zellen vieler Krebs-Patienten reagieren. Von besonderem Interesse sind aber auch solche, gegen die keine spontanen Immunantworten vorbestehen. Gegen diese können nachweislich Immunantworten induziert werden, die Tumoren lysieren können (Keogh et al, J Immunol. 167:787-96, 2001; Appella et al, Biomed Pept Proteins Nucleic Acids 1:177-84, 1995; Wentworth et al, Mol Immunol. 32:603-12, 1995).

Die offenbarten pharmazeutischen Zusammensetzungen können auch als Vacccine für die Immunisierung eingesetzt werden. Die Begriffe "Immunisierung" oder "Vaccinierung" bedeuten erfindungsgemäß eine Erhöhung oder Aktivierung einer Immunreaktion gegenüber einem Antigen. Tiermodelle können für ein Testen einer immunisierenden Wirkung gegenüber Krebs durch Verwendung eines Tumor-assoziierten Antigens oder einer dafür kodierenden Nukleinsäure eingesetzt werden. Zum Beispiel können menschliche Krebszellen in eine Maus für die Schaffung eines Tumors eingebracht werden und eine oder mehrere Nukleinsäuren, die für Tumor-assoziierte Antigene kodieren, können verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden eines oder mehrere Tumor-assoziierte Antigene oder stimulierende Fragmente davon mit einem oder mehreren Adjuvanzien für eine Induktion einer Immunreaktion oder eine Erhöhung einer Immunreaktion verabreicht. Ein Adjuvans ist eine Substanz, die in das Antigen eingebaut oder gemeinsam mit diesem verabreicht wird und die Immunreaktion verstärkt. Adjuvanzien können die Immunreaktion durch Bereitstellen eines Antigen-Reservoirs (extrazellulär oder in Makrophagen), Aktivierung von Makrophagen und Stimulierung bestimmter Lymphozyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Feundsches Adjuvans, Vitamin E, Montanid, Alaun, CpG-Oligonukleotide (vgl. Kreig et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden die Peptide in einer Mischung mit DQS21/MPL verabreicht. Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vaccine-Formulierung in einem Bereich von etwa 1 µg bis etwa 100 µg vorhanden.

Andere Stoffe, die eine Immunreaktion des Patienten stimulieren, können auch verabreicht werden. Zum Beispiel sind Cytokine bei einer Vaccinierung aufgrund ihrer regulatorischen Eigenschaften auf Lymphozyten verwendbar. Solche Cytokine umfassen z.B. Interleukin-12 (IL-12), von dem gezeigt wurde, dass es die schützenden Wirkungen von Vaccinen verstärkt (vgl. Science 268:1432-1434,1995), GM-CSF und IL-18.

Es gibt eine Reihe von Verbindungen, die eine Immunreaktion verstärken und die daher bei einer Vaccinierung eingesetzt werden können. Diese umfassen co-stimulierende Moleküle, die in Form von Proteinen oder Nukleinsäuren bereitgestellt werden. Solche costimulierenden Moleküle sind beispielsweise B7-1 und B7-2 (CD80 bzw. CD86), die auf dendritischen Zellen (DC) exprimiert werden und mit dem auf den T-Zellen exprimierten CD28-Molekül interagieren. Diese Interaktion stellt eine Co-Stimulierung (Signal 2) für eine Antigen/MHC/TCR-stimulierte (Signal 1) T-Zelle bereit, wodurch die Vermehrung der T-Zelle und die Effektorfunktion verstärkt wird. B7 interagiert auch mit CTLA4 (CD152) auf T-Zellen und Untersuchungen, die CTLA4- und B7-Liganden einbeziehen, zeigen, dass die B7-CTLA4-Interaktion eine Antitumor-Immunität und CTL-Vermehrung verstärken kann (Zheng, P. et al., Proc Natl. Acad. Sci. USA 95(11):6284-6289 (1998)).

B7 wird typischerweise nicht auf Tumorzellen exprimiert, so dass diese keine wirksamen Antigen-präsentierenden Zellen (APCs) für T-Zellen sind. Eine Induktion der B7-Expression würde ermöglichen, dass Tumorzellen wirksamer eine Vermehrung von cytotoxischen T-Lymphozyten und eine Effektorfunktion stimulieren. Eine Co-Stimulierung durch eine Kombination von B7/IL-6/IL-12 zeigte eine Induktion des IFN-gamma- und Thl-Cytokin-Profils in einer T-Zell-Population, was zu einer weiter verstärkten T-Zell-Aktivität führt (Gajewski et al., J. Immunol. 154:5637-5648 (1995)),

Eine vollständige Aktivierung von cytotoxischen T-Lymphozyten und eine vollständige Effektorfunktion erfordert eine Mitwirkung von T-Helferzellen durch die Interaktion zwischen dem CD40-Liganden auf den T-Helferzellen und dem CD40-Motekül, das von dendritischen Zellen exprimiert wird (Ridge et al., Nature 393:474 (1998), Bennett et al., Nature 393:478 (1998), Schönberger et al., Nature 393:480 (1998)). Der Mechanismus dieses co-stimulierenden Signals betrifft wahrscheinlich die Steigerung der B7- und assoziierten IL-6/IL-12-Produktion durch die dendritischen Zellen (Antigen-präsentierenden Zellen). Die CD40-CD40L-Interaktion komplementiert so die Interaktionen des Signals 1 (Antigen/MHC-TCR) und des Signals 2 (B7-CD28).

Die Verwendung von anti-CD40-Antikörpern für eine Stimulierung von dendritischen Zellen würde erwartungsgemäß direkt eine Reaktion gegenüber Tumor-Antigenen verstärken, die normalerweise außerhalb des Bereichs einer entzündlichen Reaktion liegen oder von nichtprofessionellen Antigen-präsentierenden Zellen (Tumorzellen) präsentiert werden. In diesen Situationen werden T-Helfer- und B7-co-stimulierende Signale nicht bereitgestellt. Dieser Mechanismus könnte im Zusammenhang mit Therapien verwendet werden, die auf Antigengepulsten dendritischen Zellen basieren, oder in Situationen, bei denen T-Helfer-Epitope nicht in bekannten TRA-Vorläufern definiert wurden.

Erfindungsgemäß vorgesehen ist auch eine Verabreichung von Nukleinsäuren, Polypeptiden oder Peptiden. Eine Verabreichung von Polypeptiden und Peptiden kann in an sich bekannter Weise erfolgen. In einer Ausführungsform erfolgt die Verabreichung von Nukleinsäuren durch *ex vivo*-Verfahren, d.h. durch Entfernung von Zellen aus einem Patienten, genetische Veränderung der Zellen, um ein Tumor-assoziiertes Antigen einzubauen, und Wiedereinbringung der veränderten Zellen in den Patienten. Dies umfasst im Allgemeinen das Einbringen einer funktionellen Kopie eines Gens in die Zellen eines Patienten *in vitro* und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt. Erfindungsgemäß vorgesehen ist auch eine Verabreichung von Nukleinsäuren *in vivo* durch die Verwendung von Vektoren wie Viren und zielgesteuerten Liposomen.

In einer bevorzugten Ausführungsform ist ein viraler Vektor für die Verabreichung einer Nukleinsäure, die für ein Tumor-assoziiertes Antigen kodiert, aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virus-ähnlichen Partikeln. Besonders bevorzugt sind Adenoviren und Retroviren. Die Retroviren sind üblicherweise replikationsdefizient (d.h. sie sind unfähig, infektiöse Partikel zu erzeugen).

Verschiedene Verfahren können eingesetzt werden, um erfindungsgemäß Nukleinsäuren in Zellen *in vitro* oder *in vivo* einzubringen. Solche Verfahren umfassen die Transfektion von Nukleinsäure-CaPO₄-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Bevorzugte Antikörper umfassen Antikörper, die selektiv ein Tumor-assoziiertes Antigen binden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern und ähnliches.

Die offenbarten therapeutischen Zusammensetzungen können in pharmazeutisch verträglichen Zubereitungen verabreicht werden. Solche Zubereitungen können gewöhnlich pharmazeutisch verträgliche Konzentrationen von Salzen, Pufferstoffen, Konservierungsstoffen, Trägern, ergänzenden immunitätssteigernden Stoffen wie Adjuvanzien, CpG und Cytokine und gegebenenfalls andere therapeutische Wirkstoffe enthalten.

Die offenbarten therapeutischen Wirkstoffe können auf jedem herkömmlichen Weg verabreicht werden, einschließlich durch Injektion oder durch Infusion. Die Verabreichung kann beispielsweise oral, intravenös, intraperitoneal, intramuskulär, subkutan oder transdermal erfolgen. Eine therapeutische Verabreichung von Antikörpern erfolgt vorzugsweise durch ein Lungenaerosol. Die Verabreichung von Antisense-Nukleinsäuren erfolgt vorzugsweise durch langsame intravenöse Verabreichung.

Die offenbarten Zusammensetzungen werden in wirksamen Mengen verabreicht. Eine "wirksame Menge" betrifft die Menge, die alleine oder zusammen mit weiteren Dosen eine gewünschte Reaktion oder eine gewünschte Wirkung erzielt. Im Fall einer Behandlung einer bestimmten Erkrankung oder eines bestimmten Zustands, der sich durch die Expression eines oder mehrerer Tumor-assoziierter Antigene auszeichnet, betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Krankheit oder eines Zustands kann auch die Verzögerung des Ausbruchs oder eine Verhinderung des Ausbruchs der Krankheit oder des Zustands sein.

Eine wirksame Menge einer erfindungsgemäßen Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die offenbarten pharmazeutischen Zusammensetzungen sind vorzugsweise steril und enthalten eine wirksame Menge der therapeutisch wirksamen Substanz für die Erzeugung der gewünschten Reaktion oder der gewünschten Wirkung.

Die Dosen der offenbarten Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunreaktion Dosen des Tumor-assoziierten Antigens von 1 ng bis 1 mg, vorzugsweise von 10 ng bis 100 µg formuliert und verabreicht. Falls die Verabreichung von Nukleinsäuren (DNA sowie RNA), die für Tumor-assoziierte Antigene kodieren, erwünscht ist, werden Dosen von 1 ng bis 0,1 mg formuliert und verabreicht.

Die vorliegend offenbarten pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nichttoxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff , Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine pharmazeutische Zusammensetzung kann einen pharmazeutisch verträglichen Träger umfassen. Der Begriff "pharmazeutisch verträglicher Träger" betrifft erfindungsgemäß einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der erfindungsgemäßen pharmazeutischen Zusammensetzung sind gewöhnlich derart, dass keine Interaktion auftritt, die die gewünschte pharmazeutische Wirksamkeit wesentlich beeinträchtigt.

Die pharmazeutischen Zusammensetzungen können geeignete Pufferstoffe wie Essigsäure in einem Satz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Erfindungsgemäße pharmazeutische Zusammensetzungen können beispielsweise in Form von Kapseln, Tabletten, Lutschpastillen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich.

### Abbildungen:

**Abb. 1****: Schematische Darstellung der Klonierung von eCT.** Die Strategie umfasst die Identifizierung von Kandidaten-Genen (GOI = "Genes of interest") in Datenbanken und das Testen dieser Gene durch RT-PCR.

**Abb. 2****: RT-PCR für BRCO1.** In Brusttumoren ist **BRCO1** im Vergleich zu der Expression in normalem Brustdrüsengewebe deutlich überexprimiert.

### Beispiele:

### Material und Methoden

Die Begriffe "*in silico*", "elektronisch" und "virtuell klonieren" beziehen sich rein auf die Nutzung von auf Datenbanken beruhenden Verfahren, mit denen auch Labor-experimentelle Vorgänge simuliert werden können.
Alle anderen Begriffe und Termini sind, falls nicht explizit anders definiert, so verwendet, wie sie der Fachmann versteht. Die genannten Techniken und Methoden erfolgen in an sich bekannter Weise und sind z.B. in Sambrook et al., Molecular Cloning: A Laboratory Manual, 2. Auflage (1989) Cold Spring Harbor Laboratory Press, Cold Spring Harbor, N. Y beschrieben. Alle Verfahren, die die Verwendung von Kits und Reagenzien einschließen, sind entsprechend den Angaben der Hersteller durchgeführt.

### Datamining-basierte Strategie zur Ermittlung von eCT (elektronisch klonierte Cancer/Testis-Gene)

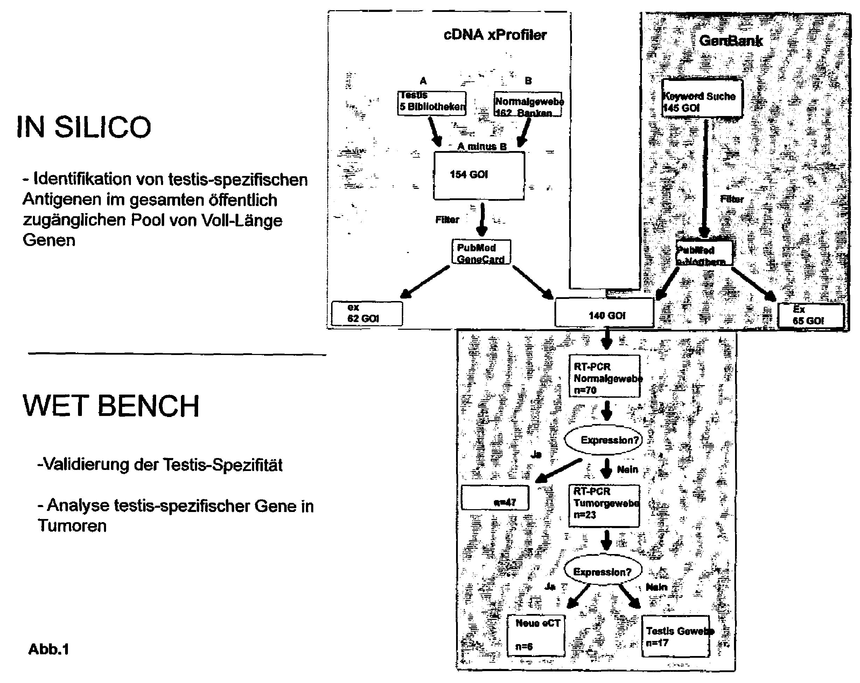

Zwei *in silico* Strategien nämlich GenBank-Schlagwort-Suche und der cDNAxProfiler wurden kombiniert (Abb. 1). Es wurde unter Nutzung des ENTREZ Search and Retrieval Systems des NCBI (http://www.ncbi.nlm.nih.gov/Entrez) eine Suche nach Kandidaten-Genen in der GenBank durchgeführt., die annotiert sind als spezifisch exprimiert in testikulärem Gewebe (Wheeler et al., Nurcleic Acids Research 28:10-14, 2000).
Durch Suchabfragen mit den Schlagworten "testis-specific gene", "sperm-specific gene", "spermatogonia-specific gene" wurden Kandidatengene (GOI, genes of interest) aus den Datenbanken herausextrahiert. Die Suche wurde auf einen Teil der Gesamtinformation dieser Datenbanken eingeschränkt, indem als Limits "homo sapiens" für den Organismus und "mRNA" für die Molekülart eingesetzt wurden.

Die Liste der gefundenen GOI wurde kuratiert, indem unterschiedliche Bezeichnungen für dieselbe Sequenz ermittelt und solche Redundanzen behoben wurden.
Alle Kandidatengene, die sich durch die Schlagwort-Suche ergaben, wurden wiederum durch das Verfahren des "electronic Northern" (eNorthern) bezüglich ihrer Gewebeverteilung untersucht. Der eNorthern basiert darauf, dass die Sequenz eines GOI gegenüber einer EST-(expressed sequence tag) Datenbank (Adams et al., Science 252:1651,1991) abgeglichen wird (http://www.ncbi.nlm.nih.gov/BLAST). Zu jedem EST, das sich als homolog zum eingegebenen GOI ergibt, lässt sich die Gewebeherkunft ermitteln und durch die Summe aller ESTs auf diese Weise eine vorläufige Einschätzung der Gewebeverteilung des GOI erreichen. Nur diejenigen GOI wurden weiteren Untersuchungen zugeführt, die keine Homologien zu EST aus nicht-testikulären Normalgeweben mit Ausnahme von Plazenta und fetalem Gewebe hatten. Für dies Beurteilung wurde auch berücksichtigt, dass es falsch-annotierte cDNA Banken in der öffentlichen Domäne gibt (Scheurle et al., Cancer Res. 60:4037-4043, 2000) (www.fau.edu/cmbb/publications/cancergenes6.htm).
Als zweites Datamining-Verfahren wurde der **cDNA xProfiler** des Cancer Genome Anatomy Projekts des NCBI (http://cgap.nci.nih.gov/Tissues/xProfiler) genutzt (Hillier et al., Genome Research 6:807-828, 1996; Pennisi, Science 276:1023-1024, 1997). Dieser erlaubt Pools von in Datenbanken abgelegten Transkriptomen durch logische Operatoren in Beziehung zueinander zu setzen. Wir haben einen Pool A definiert, dem alle aus Testis hergestellten Expressionsbibliotheken, unter Ausschluss von gemischten Bibliotheken zugeordnet wurden. Dem Pool B wurden alle cDNA-Bibliotheken zugeordnet, die von Normalgeweben mit Ausnahme von Testis, Ovar und Fetalgewebe hergestellt waren. Generell wurden alle cDNA-Banken unabhängig vom zugrundeliegenden Herstellungsverfahren genutzt, allerdings lediglich solche mit einer Mächtigkeit > 1000 zugelassen. Mittels des BUT NOT Operators wurde Pool B digital von Pool A subtrahiert. Auch das Set der auf diese Weise gefundenen GOI wurde eNorthem-Studien unterzogen, sowie durch eine Literaturrecherche abgesichert. Dieses kombinierte Datamining schließt alle etwa 13 000 Vollänge-Gene in der öffentlichen Domäne ein und prädiziert aus diesen insgesamt 140 Gene mit potentieller Testis-spezifischer Expression. Unter diesen waren 25 bereits bekannte Gene der CT-Genklasse, was die Effizienz unserer Strategie unterstreicht.
Alle anderen Gene wurden zunächst durch spezifische RT-PCR in Normalgeweben evaluiert. Alle GOI, die sich als in nicht-testikulären Normalgeweben exprimiert erwiesen, hatten als Falsch-Positive zu gelten und wurden aus weiteren Untersuchungen ausgeschlossen. Die verbliebenen wurden in einem großen Panel an verschiedensten Tumorgeweben untersucht. Die unten dargestellten Antigene erwiesen sich dabei als ektop in Tumorzellen aktiviert.

### RNA-Extraktion, Herstellung von poly-d(T) geprimter cDNA und RT-PCR Analyse

Gesamt-RNA aus nativem Gewebematerial wurde unter Verwendung von Guanidiumisothiocyanate als chaotrophem Agens extrahiert (Chomczynski & Sacchi, Anal. Biochem. 162:156-9, 1987). Nach Extraktion mit saurem Phenol und Fällung mit Isopropanol wurde die RNA in DEPC-behandeltem Wasser gelöst.
Aus 2-4 µg Gesamt-RNA wurde in einem 20µl Reaktionsansatz mittels Superscript Il (Invitrogen) entsprechend den Angaben des Herstellers eine Erststrang-cDNA-Synthese durchgeführt. Als Primer wurde ein dT(18) Oligonukleotid verwendet. Integrität und Qualität der cDNA wurden durch Amplifikation von p53 in einer 30 Zyklen-PCR (sense CGTGAGCGCTTCGAGATGTTCCG, antisense CCTAACCAGCTGCCCAACTGTAG, Hybridisierungstemperatur 67°C) überprüft.
Es wurde ein Archiv aus Erststrang-cDNAs aus einer Reihe von Normalgeweben und Tumorentitäten hergestellt. Für Expressionsstudien wurden 0,5 µl dieser cDNAs in einem 30µl Reaktionsansatz mit GOI-spezifischen Primern (siehe unten) und 1 U HotStarTaq DNA Polymerase (Qiagen) amplifiziert. Der Reaktionsansatz enthielt jeweils 0,3 mM dNTPs, je 0,3 µM jeden Primers und 3 µl 10 x Reaktionspuffer.
Die Primer wurden so ausgewählt, dass sie in 2 verschiedenen Exons liegen und die Beseitigung der Interferenz durch kontaminierende genomische DNA als Grund für falsch positive Resultate wurde durch Testen von nicht revers transkribierter DNA als Matritze bestätigt. Nach 15 Minuten bei 95°C zur Aktivierung der HotStarTaq DNA Polymerase wurden 35 Zyklen PCR durchgeführt (1 min 94°C, 1 min jeweilige Hybridisierungstemperatur, 2 min 72°C und abschließende Elongation bei 72°C für 6 min).
20µl dieser Reaktion wurden auf einem mit Ethidiumbromid gefärbten Agarosegel aufgetrennt und analysiert.

Folgende Primer wurden für die Expressionsanalyse von BRCO1 bei der angegebenen Hybridisierungstemperatur verwendet:
BRCO1 (60°C)
   sense CTTGCTCTGAGTCATCAGATG, antisense CACAGAATATGAGCCATACAG

### Herstellung von Random-Heaamer-geprimter cDNA und quantitative Real-Time-PCR

Die Expression von LDHC wurde mittels Real-Time-PCR quantifiziert.
Das Prinzip der quantitativen Real-Time-PCR mit dem ABI PRISM Sequence Detection System (PE Biosystems, USA) nutzt die 5'-3' Exonuklease-Aktivität der Taq DNA Polymerase zur direkten und spezifischen Detektion von PCR-Produkten durch Freisetzung von Fluoreszenz-Reporterfarbstoffen. Neben Sense- und Antisense-Primern wird bei der PCR eine doppelt fluoreszenz-markierte Sonde (TaqMan-Probe) eingesetzt die an eine Sequenz des PCR-Produkts hybridisiert. Die Sonde ist 5' mit einem Reporterfarbstoff (z.B. FAM) und 3' mit einem Quencherfarbstoff (z.B. TAMRA) markiert. Ist die Sonde intakt, supprimiert die räumliche Nähe von Reporter zu Quencher die Emission der Reporterfluoreszenz. Hybridisiert die Sonde während der PCR an das PCR-Produkt, wird sie durch die 5'-3' Exonuklease-Aktivität der Taq DNA Polymerase gespalten und die Suppression der Reporterfluorszenz aufgehoben. Das Ansteigen der Reporterfluoreszenz als Folge der Zielamplifikation wird nach jedem PCR-Zyklus gemessen und zur Quantifizierung genutzt. Die Expressionsquantifizierung des Zielgens erfolgt absolut oder relativ zur Expression eines Kontrollgens mit konstanter Expression in den zu untersuchenden Geweben. Die Expression von LDHC wurde nach Normalisierung der Proben gegen 18s RNA als sog. Housekeeping-Gen mittels der ΔΔ-Cₜ Methode (PE Biosystems, USA) berechnet. Die Reaktionen wurden in Duplex-Ansätzen durchgeführt und in Duplikaten bestimmt. Die Synthese der cDNA erfolgte mit dem High Capacity cDNA Archive Kit (PE Biosystems, USA) unter Verwendung von Hexamer-Primern nach Angaben des Herstellers. Jeweils 5 µl der verdünnten cDNA wurden in 25 µl Gesamtvolumen für die PCR eingesetzt: sense-Primer (GGTGTCACTTCTGTGCCTTCCT) 300 nM; antisense-Primer (CGGCACCAGTTCCAACAATAG) 300 nM; TaqMan-Probe (CAAAGGTTCTCCAAATGT) 250 nM; sense-Primer 18s RNA 50 nM; antisense-Primer 18s RNA 50 nM; 18 s RNA-Probe 250 nM; 12,5 µl TaqMan Universal PCR Master Mix; anfängliche Denaturierung 95°C (10 min); 95°C (15 sec); 60°C (1 min); 40 Zyklen. Durch die Amplifikation eines 128 bp-Produkts über die Grenze von Exon 1 und Exon 2 wurden alle beschriebenen Splicevarianten von LDHC in die Quantifizierung einbezogen.

### Klonierung und Sequenzanalyse

Klonierung von Vollängen bzw. Genfragmenten erfolgte nach gängigen Methoden. Zur Ermittlung der Sequenz wurden entsprechende Antigene mittels der Proofreading-Polymerase pfu (Stratagene) amplifiziert. Nach Beendigung der PCR wurde Adenosin mittels HotStarTaq DNA Polymerase an die Enden des Amplikons ligiert, um die Fragmente entsprechend den Angaben des Herstellers in den TOPO-TA Vektor zu klonieren. Die Sequenzierung wurde durch einen kommerziellen Service durchgeführt. Die Sequenzen wurden mittels gängiger Prädiktionsprogramme und Algorithmen analysiert.

### Beispiel 1: Identifizierung von BRCO1 als neues Tumorantigen

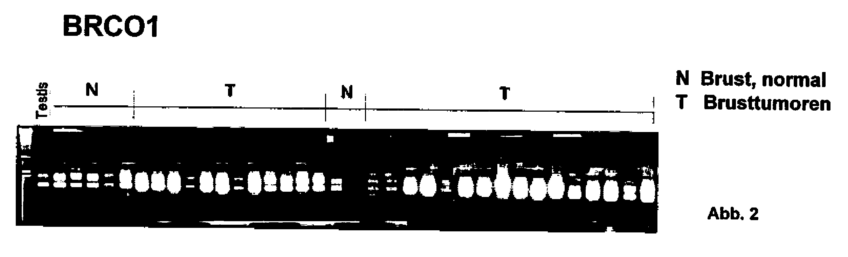

BRCO1 und sein Translationsprodukt sind nicht vorbeschrieben. Das erfindungsgemäße Datamining-Verfahren ergab das EST (expressed sequence tag) AI668620. Mit spezifischen Primern (sense: CTTGCTCTGAGTCATCAGATG (SEQ ID NO: 46), antisense: CACAGAATATGAGCCATACAG (SEQ ID NO: 47)) wurden RT-PCR Studien zur Expressionsanalyse durchgeführt. Erfindungsgemäß wurde spezifische Expression in testikulärem Gewebe, sowie zusätzlich in normaler Brustdrüse gefunden (Tabelle 1). In allen anderen Geweben ist dieses Antigen transkriptionell reprimiert. In Brustdrüsentumoren wird es ebenfalls (20 von 20) nachgewiesen. In Brusttumoren ist BRCO1 im Vergleich zu der Expression in normalem Brustdrüsengewebe deutlich überexprimiert (Abb. 2).
Durch elektronische Vollängeklonierung unter Nutzung von EST-contigs (folgende EST wurden inkorporiert AW137203, BF327792, BF327797, BE069044, BF330665) gelang erfindungsgemäß die Klonierung von mehr als 1500 bp dieses Transkriptes (SEQ ID NO:39). Die Sequenz kartiert auf Chromosom 10p11-12. In der gleichen Region in unmittelbarer Nähe ist bereits das Gen für ein Mamma-DifferenzierungsAntigen NY-BR-1 beschrieben worden (NM_052997; Jager, D. et al., Cancer Res. 61(5):2055-61, 2001). WO01/51628 offenbart zahlreiche Nukleotidsequenzen, einschließlich der als "SEQ ID NO: 2898" und "SEQ ID NO: 16804" bezeichneten Nukleotidsequenzen, die kürzere, vollständig identische Teilbereiche von SEQ ID NO: 39 der vorliegenden Erfindung enthalten. Ebenso offenbart WO01/60860 eine als "cDNA 20306" bezeichnete Nukleotidsequenz, die einen kürzeren, identischen Teilbereich von SEQ ID NO: 39 enthält. Die Nukleotidsequenz gemäß SEQ ID NO: 39 der vorliegenden Erfindung wird jedoch als solche im Stand der Technik nicht beschrieben.

**Tabelle 1. Expression von BRCO1 in Normalgeweben und Brusttumoren**

| | |
|---|---|
| **Ileum** | - |
| **Kolon** | - |
| **Leber** | - |
| **Lunge** | - |
| **Lymphknoten** | - |
| **Magen** | - |
| **Milz** | - |
| **Nebenniere** | - |
| **Niere** | - |
| **Ösophagus** | - |
| **Ovar** | - |
| **Rektum** | - |
| **Testis** | + |
| **Thymus** | - |
| **Haut** | - |
| **Mamma** | + |
| **Pankreas** | - |
| **PBMC** | - |
| **PBMC akt.** | - |
| **Prostata** | - |
| **Schilddrüse** | - |
| **Tube** | - |
| **Uterus** | - |
| **Grosshirn** | - |
| **Kleinhirn** | - |
| **Mammakarzinome** | ++ **(20/20)** |

In matched pair (Mama-Karzinom und benachbartes Normalgewebe) Untersuchungen zeigte sich erfindungsgemäß in 70 % der Mama-Karzinome eine BRCO1 Überexpression im Vergleich zum Normagewebe.

Somit ist BRCO1 ein neues DifferenzierungsAntigen für normales Brustdrüsenepithel, das in Brusttumoren überexprimiert wird.

### SEQUENCE LISTING

<114> Sahin Dr., Ugur
   Türeci Dr., Özlem
   Koslowski Dr., Michael
<120> Differentiell in Tumoren exprimierte Genprodukte und deren Verwendung
<130> 342-3 EPT11
<160> 3
<170> PatentIn version 3.1
<210> 39
   <211> 1948
   <212> DNA
   <213> Homo sapiens
<400> 39
   gcacgaggtt ttgaggacca gcaacacagc aatacttcca gatctccata taacctctgt      60
   tcatttggga ggggctttgt attttcaaca ggagagttca aagttcattt ttttttcagc      120
   aactacagtt ctaagtgaaa tctattttta ttgatacatg gtattttaca tgtttatggg     180
   atacatatga gtcataatct attttaaata ataccttagt gttgtaaaat caacagtgct     240
   ttttaaaaga aatatacctt gttaattatc ccacatgtgt ctccagaagt acagcttgaa     300
   caaatccacc ttctgtggac caagcaccac cctgggcatt tctagcatga gcaaaatcca     360
   aggtcctggc tggactccag agatgctatt tacctcagaa gcatgacaat aggaggcaga     420
   aggagcaggc aaatccaagt cctttcttgt agtttccttg tttggggagg aaaagttgag     480
   ttttactatt atggaaaaga aacaggaaat agagacagac aaagagatat gacaatacag     540
   tcctgccacc cagatactca tttccaccta ccattccatg catttgtttt gaatatataa     600
   gtatgtacat aaaggtaggt actctcaagt ccatcagggc ttggctgtcc actgtttttg     660
   aagttccaga atgtttttgc taagttgagg aaataccaaa tcaggactat gaaaattatg     720
   gtatatattg atgtgtcaca gaacacagat gtgacataat aaagatgtgt aagattatat     780
   atataacttg tgtgtacacc tacctcatct ggggataaca cctcaagttt aattttgagg     840
   cttgggtcaa tcgtgcttcc cttccctttc ataggtcctc tatgagatat tgtcatagat     900
   tccatgttat gcaatagcca tagaatatga catctctcta tgataattct atattacttt     960
   aattgctgca cagaagttca ttgtatgtaa gtgccacagt atattataga tcttcttgtg    1020
   ggacatctat ttctagttta tgtgatagta tagcactttc atgaatgttc ttgtacttga    1080
   tctttacaca ttttcttttt tccttaggat gaattctgag agatgtaatt gatggggcaa    1140
   aatgtactca ctgtttgagg tttgaaattt ttccatcaaa agctggtact cttggttttt    1200
   taagacaaag agcaaatcct cccctgccag gattgacttt tggctctttt ttttcaaacc 1260
   tcactgcttt ttggtttagt tgtcataaaa tgccaagcac catgaacagg gctccatgaa 1320
   ggggctcaga ggtaggaggg ctgtgattag gagaaggctt ggactgatgg gcaatttgag 1380
   tgctcagaat tagagtgagg gggtgggggt gctgcaggga cagatgctgg ggaaagacac 1440
   cctgaagggc aaagggagca acaatggctg cagtacatgt ggcctttcag ctagcgcaga 1500
   ggatggaaac cagagtgggc tgatgattgg atgccaggcc tgagccagca actgtgatcc 1560
   tgagctgtgc acacttctgg ttgggattat ttctggtttc tacttcctgt ttgaagatgt 1620
   ggcatggaga gtgctctgct ttgacctgaa gtattttatc tatcctcagt ctcaggacac 1680
   tgttgatgga attaaggcca agcacatctg caaaaaagac attgctggag gaggtgcaaa 1740
   gagctggaaa ccaagtctcc agtcctggga aaagcagtgg tatggaaaag caatggaaag 1800
   agcattttga aaatgccatt ccactgtttt ctggccttta tgatttctgc tgagaaatcc 1860
   actgttagtc tgatggggtc tccttcatag caccaatgac ctgaagagcc ttgttgaagg 1920
   aagactccat ctgatgactc agagcaag 1948
<210> 46
   <211> 21
   <212> DNA
   <213> Künstliche Sequenz
<400> 46
   cttgctctga gtcatcagat g 21
<210> 47
   <211> 21
   <212> DNA
   <213> Künstliche Sequenz
<400> 47
   cacagaatat gagccataca g 21

## Patentansprüche

1. Verfahren zur Diagnose einer Krebserkrankung, die sich durch die Expression oder abnormale Expression eines Tumor-assoziierten Antigens auszeichnet, umfassend
(i) den Nachweis einer Nukleinsäure, die für das Tumor-assoziierte Antigen kodiert, und/oder
(ii) den Nachweis des Tumor-assoziierten Antigens,
in einer aus einem Patienten isolierten biologischen Probe,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 39 umfasst,
(b) einer Nukleinsäure, die in Bezug auf die Nukleinsäure unter (a) degeneriert ist und
(c) einer Nukleinsäure, die zu der Nukleinsäure unter (a) oder (b) eine Sequenzidentität von mindestens 90% aufweist.

2. Verfahren zur Bestimmung der Regression, des Verlaufs oder des Ausbruchs einer Krebserkrankung, die sich durch die Expression oder abnormale Expression eines Tumor-assoziierten Antigens auszeichnet, umfassend die Überwachung einer Probe aus einem Patienten, der die Erkrankung aufweist oder in Verdacht steht, an der Erkrankung zu erkranken, in Bezug auf einen oder mehrere Parameter, ausgewählt aus der Gruppe, bestehend aus:
(i) der Menge der Nukleinsäure, die für das Tumor-assoziierte Antigen kodiert, und
(ii) der Menge des Tumor-assoziierten Antigens,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 39 umfasst,
(b) einer Nukleinsäure, die in Bezug auf die Nukleinsäure unter (a) degeneriert ist und
(c) einer Nukleinsäure, die zu der Nukleinsäure unter (a) oder (b) eine Sequenzidentität von mindestens 90% aufweist.

3. Antikörper, der spezifisch an ein Protein oder Polypeptid bindet, wobei das Protein oder Polypeptid von einer Nukleinsäure kodiert wird, die aus der Gruppe ausgewählt ist, bestehend aus:
(a) einer Nukleinsäure, die eine Nukleinsäuresequenz gemäß SEQ ID NO: 39 umfasst,
(b) einer Nukleinsäure, die in Bezug auf die Nukleinsäure unter (a) degeneriert ist und
(c) einer Nukleinsäure, die zu der Nukleinsäure unter (a) oder (b) eine Sequenzidentität von mindestens 90% aufweist.

4. Antikörper nach Anspruch 3, wobei der Antikörper mit einem diagnostischen Mittel gekoppelt ist.

5. Antikörper nach Anspruch 4 zur Verwendung in einem Verfahren zur Diagnose oder Überwachung einer Krebserkrankung, die sich durch die Expression oder abnormale Expression eines Tumor-assoziierten Antigens auszeichnet, wobei das Verfahren die Verabreichung des Antikörpers umfasst und das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 39 umfasst,
(b) einer Nukleinsäure, die in Bezug auf die Nukleinsäure unter (a) degeneriert ist und
(c) einer Nukleinsäure, die zu der Nukleinsäure unter (a) oder (b) eine Sequenzidentität von mindestens 90% aufweist.

6. Antikörper nach Anspruch 3 oder 4 oder Antikörper zur Verwendung nach Anspruch 5, wobei der Antikörper ein monoklonaler, chimärer oder humanisierter Antikörper oder ein Fragment eines Antikörpers ist.

7. Verfahren nach Anspruch 1 oder 2 oder Antikörper zur Verwendung nach Anspruch 5 oder 6, wobei die Krebserkrankung ein Lungentumor, ein Brusttumor, ein Prostatatumor, ein Melanom, ein Colontumor, eine Metastase eines Colontumors, ein Nierenzellkarzinom, ein Zervixkarzinom, ein Coloncarcinom oder ein Mammacarcinom ist.

8. Nukleinsäure, die aus der Gruppe ausgewählt ist, bestehend aus:
(a) einer Nukleinsäure, die eine Nukleinsäuresequenz gemäß SEQ ID NO: 39 umfasst,
(b) einer Nukleinsäure, die in Bezug auf die Nukleinsäure unter (a) degeneriert ist und
(c) einer Nukleinsäure, die zu der Nukleinsäure unter (a) oder (b) eine Sequenzidentität von mindestens 90% aufweist.

9. Nukleinsäure die zu der Nukleinsäure gemäß Anspruch 8 komplementär ist und mindestens 90% Sequenzidentität aufweist.

10. Oligonukleotid oder Polynukleotid, das unter stringenten Bedingungen spezifisch mit einer Nukleinsäure hybridisiert, die eine Nukleinsäuresequenz gemäß SEQ ID NO: 39 umfasst.

11. Verwendung der Nukleinsäure gemäß Anspruch 9 oder des Oligonukleotids oder Polynukleotids nach Anspruch 10 zur Diagnose oder Überwachung einer Krebserkrankung, die sich durch die Expression eines Tumor-assoziierten Antigens auszeichnet, wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 39 umfasst,
(b) einer Nukleinsäure, die in Bezug auf die Nukleinsäure unter (a) degeneriert ist und
(c) einer Nukleinsäure, die zu der Nukleinsäure unter (a) oder (b) eine Sequenzidentität von mindestens 90% aufweist.

12. Wirtszelle, die eine Nukleinsäure nach Anspruch 8 umfasst.

13. Protein oder Polypeptid, das von einer Nukleinsäure nach Anspruch 8 kodiert wird.

14. Verwendung eines Kits zur Diagnose einer Krebserkrankung, die sich durch die Expression oder abnormale Expression eines Tumor-assoziierten Antigens auszeichnet, umfassend Mittel zum Nachweis
(i) der Nukleinsäure, die für das Tumor-assoziierte Antigen kodiert,
(ii) des Tumor-assoziierten Antigens,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 39 umfasst,
(b) einer Nukleinsäure, die in Bezug auf die Nukleinsäure unter (a) degeneriert ist und
(c) einer Nukleinsäure, die zu der Nukleinsäure unter (a) oder (b) eine Sequenzidentität von mindestens 90% aufweist.

## Claims

1. A method for diagnosing a cancerous disease **characterized by** expression or abnormal expression of a tumor-associated antigen, which method comprises
(i) detecting a nucleic acid which codes for the tumor-associated antigen, and/or
(ii) detecting the tumor-associated antigen,
in a biological sample isolated from a patient,
wherein the tumor-associated antigen has a sequence which is encoded by a nucleic acid selected from the group consisting of:
(a) a nucleic acid which comprises a nucleic acid sequence according to SEQ ID NO: 39,
(b) a nucleic acid which is degenerate with respect to the nucleic acid of (a), and
(c) a nucleic acid which has a sequence identity of at least 90% to the nucleic acid of (a) or (b).

2. A method for determining regression, course or onset of a cancerous disease **characterized by** expression or abnormal expression of a tumor-associated antigen, which method comprises monitoring a sample from a patient who has the disease or is suspected of falling ill with the disease with respect to one or more parameters selected from the group consisting of:
(i) the amount of the nucleic acid which codes for the tumor-associated antigen, and
(ii) the amount of the tumor-associated antigen,
wherein the tumor-associated antigen has a sequence which is encoded by a nucleic acid selected from the group consisting of:
(a) a nucleic acid which comprises a nucleic acid sequence according to SEQ ID NO: 39,
(b) a nucleic acid which is degenerate with respect to the nucleic acid of (a), and
(c) a nucleic acid which has a sequence identity of at least 90% to the nucleic acid of (a) or (b).

3. An antibody which binds specifically to a protein or polypeptide, wherein the protein or polypeptide is encoded by a nucleic acid selected from the group consisting of:
(a) a nucleic acid which comprises a nucleic acid sequence according to SEQ ID NO: 39,
(b) a nucleic acid which is degenerate with respect to the nucleic acid of (a), and
(c) a nucleic acid which has a sequence identity of at least 90% to the nucleic acid of (a) or (b).

4. The antibody according to claim 3, wherein the antibody is linked to a diagnostic agent.

5. The antibody according to claim 4 for use in a method for diagnosing or monitoring a cancerous disease **characterized by** expression or abnormal expression of a tumor-associated antigen, wherein the method comprises administering the antibody and the tumor-associated antigen has a sequence which is encoded by a nucleic acid selected from the group consisting of:
(a) a nucleic acid which comprises a nucleic acid sequence according to SEQ ID NO: 39,
(b) a nucleic acid which is degenerate with respect to the nucleic acid of (a), and
(c) a nucleic acid which has a sequence identity of at least 90% to the nucleic acid of (a) or (b).

6. The antibody according to claim 3 or 4 or antibody for use according to claim 5, wherein the antibody is a monoclonal, chimeric or humanized antibody or a fragment of an antibody.

7. The method according to claim 1 or 2 or antibody for use according to claim 5 or 6, wherein the cancerous disease is a lung tumor, a breast tumor, a prostate tumor, a melanoma, a colon tumor, a metastasis of a colon tumor, a renal cell carcinoma, a cervical carcinoma, a colon carcinoma or a mammary carcinoma.

8. A nucleic acid selected from the group consisting of:
(a) a nucleic acid which comprises a nucleic acid sequence according to SEQ ID NO: 39,
(b) a nucleic acid which is degenerate with respect to the nucleic acid of (a), and
(c) a nucleic acid which has a sequence identity of at least 90% to the nucleic acid of (a) or (b).

9. A nucleic acid which is complementary and has at least 90% sequence identity to the nucleic acid according to claim 8.

10. An oligonucleotide or a polynucleotide, which hybridizes under stringent conditions specifically with a nucleic acid comprising a nucleic acid sequence according to SEQ ID NO: 39.

11. Use of the nucleic acid according to claim 9 or of the oligonucleotide or polynucleotide according to claim 10 for diagnosing or monitoring a cancerous disease **characterized by** expression of a tumor-associated antigen, wherein the tumor-associated antigen has a sequence which is encoded by a nucleic acid selected from the group consisting of:
(a) a nucleic acid which comprises a nucleic acid sequence according to SEQ ID NO: 39,
(b) a nucleic acid which is degenerate with respect to the nucleic acid of (a), and
(c) a nucleic acid which has a sequence identity of at least 90% to the nucleic acid of (a) or (b).

12. A host cell comprising a nucleic acid according to claim 8.

13. A protein or polypeptide encoded by a nucleic acid according to claim 8.

14. Use of a kit for diagnosing a cancerous disease **characterized by** expression or abnormal expression of a tumor-associated antigen comprising means for detecting
(i) the nucleic acid which codes for the tumor-associated antigen,
(ii) the tumor-associated antigen,
wherein the tumor-associated antigen has a sequence which is encoded by a nucleic acid selected from the group consisting of:
(a) a nucleic acid which comprises a nucleic acid sequence according to SEQ ID NO: 39,
(b) a nucleic acid which is degenerate with respect to the nucleic acid of (a), and
(c) a nucleic acid which has a sequence identity of at least 90% to the nucleic acid of (a) or (b).

## Revendications

1. Procédé pour le diagnostic d'une affection cancéreuse qui se manifeste par l'expression ou l'expression anormale d'un antigène associé à une tumeur, comprenant
(i) la mise en évidence d'un acide nucléique qui code pour l'antigène associé à une tumeur, et/ou
(ii) la mise en évidence de l'antigène associé à une tumeur,
dans un échantillon biologique isolé à partir d'un patient,
tandis que l'antigène associé à une tumeur présente une séquence qui est codée par un acide nucléique qui est choisi dans le groupe consistant en:
(a) un acide nucléique qui comprend une séquence d'acide nucléique selon SEQ ID NO: 39,
(b) un acide nucléique qui est dégénéré par rapport à l'acide nucléique selon (a), et
(c) un acide nucléique qui présente une identité de séquences d'au moins 90% par rapport à l'acide nucléique selon (a) ou (b).

2. Procédé pour la détermination de la régression, de l'évolution ou de l'interruption d'une affection cancéreuse qui se manifeste par l'expression ou l'expression anormale d'un antigène associé à une tumeur, comprenant le contrôle d'un échantillon provenant d'un patient qui présente l'affection ou qui est susceptible d'être atteint par l'affection, en ce qui concerne un ou plusieurs paramètres, choisis dans le groupe consistant en:
(i) la quantité de l'acide nucléique qui code pour l'antigène associé à une tumeur, et
(ii) la quantité de l'antigène associé à une tumeur,
tandis que l'antigène associé à une tumeur présente une séquence qui est codée par un acide nucléique qui est choisi dans le groupe consistant en:
(a) un acide nucléique qui comprend une séquence d'acide nucléique selon SEQ ID NO: 39,
(b) un acide nucléique qui est dégénéré en ce qui concerne l'acide nucléique selon (a) et
(c) un acide nucléique qui présente une identité de séquences d'au moins 90% par rapport à l'acide nucléique selon (a) ou (b).

3. Anticorps qui se lie spécifiquement à une protéine ou un polypeptide, tandis que la protéine ou le polypeptide est codé par un acide nucléique qui est choisi dans le groupe consistant en:
(a) un acide nucléique qui comprend une séquence d'acide nucléique selon SEQ ID NO: 39,
(b) un acide nucléique qui est dégénéré en ce qui concerne l'acide nucléique selon (a), et
(c) un acide nucléique qui présente une identité de séquences d'au moins 90% par rapport à l'acide nucléique selon (a) ou (b).

4. Anticorps selon la revendication 3, dans lequel l'anticorps est couplé avec un moyen de diagnostic.

5. Anticorps selon la revendication 4 pour utilisation dans un procédé pour le diagnostic ou le suivi d'une affection cancéreuse qui se manifeste par l'expression ou l'expression anormale d'un antigène associé à une tumeur, tandis que le procédé comprend l'administration de l'anticorps et l'antigène associé à la tumeur présente une séquence qui est codée par un acide nucléique qui est choisi dans le groupe consistant en:
(a) un acide nucléique qui comprend une séquence d'acide nucléique selon SEQ ID NO: 39,
(b) un acide nucléique qui est dégénéré par rapport à l'acide nucléique selon (a), et
(c) un acide nucléique qui présente, par rapport à l'acide nucléique sous (a) ou (b), une identité de séquences d'au moins 90%.

6. Anticorps selon la revendication 3 ou 4 ou anticorps pour utilisation selon la revendication 5, dans lesquels l'anticorps est un anticorps ou un fragment d'anticorps monoclonal, chimérique ou humanisé.

7. Procédé selon l'une des revendications 1 ou 2 ou anticorps pour utilisation selon la revendication 5 ou 6, tandis que l'affection cancéreuse est une tumeur pulmonaire, une tumeur du sein, une tumeur de la prostate, un mélanome, une tumeur du côlon, une métastase d'une tumeur du côlon, un carcinome des cellules rénales, un carcinome du col de l'utérus, un carcinome du côlon ou un carcinome mammaire.

8. Acide nucléique, qui est choisi dans le groupe consistant en:
(a) un acide nucléique qui comprend une séquence d'acide nucléique selon SEQ ID NO: 39,
(b) un acide nucléique qui est dégénéré par rapport à l'acide nucléique selon (a), et
(c) un acide nucléique qui présente par rapport à l'acide nucléique selon (a) ou (b) une identité de séquences d'au moins 90%.

9. Acide nucléique qui par rapport à l'acide nucléique selon la revendication 8 est complémentaire et présente au moins 90% d'identité de séquences.

10. Oligonucléotide ou polynucléotide, qui hybride spécifiquement dans des conditions stringentes avec un acide nucléique qui comprend une séquence d'acide nucléique selon SEQ ID NO: 39.

11. Utilisation de l'acide nucléique selon la revendication 9 ou de l'oligonucléotide ou polynucléotide selon la revendication 10 pour le diagnostic ou le suivi d'une affection cancéreuse qui se manifeste par l'expression d'un antigène associé à une tumeur, tandis que l'antigène associé à la tumeur présente une séquence qui est codée par un acide nucléique qui est choisi dans le groupe consistant en:
(a) un acide nucléique qui comprend une séquence d'acide nucléique selon SEQ ID NO: 39,
(b) un acide nucléique qui est dégénéré par rapport à l'acide nucléique selon (a), et
(c) un acide nucléique qui présente, par rapport à l'acide nucléique sous (a) ou (b), une identité de séquences d'au moins 90%.

12. Cellule hôte, qui comprend un acide nucléique selon la revendication 8.

13. Protéine ou polypeptide, qui est codé par un acide nucléique selon la revendication 8.

14. Utilisation d'un kit pour le diagnostic d'une affection cancéreuse qui se manifeste par l'expression ou l'expression anormale d'un antigène associé à une tumeur, comprenant des moyens pour la mise en évidence
(i) de l'acide nucléique qui code pour l'antigène associé à une tumeur,
(ii) de l'antigène associé à une tumeur,
tandis que l'antigène associé à une tumeur présente une séquence qui est codée par un acide nucléique qui est choisi dans le groupe consistant en:
(a) un acide nucléique qui comprend une séquence d'acide nucléique selon SEQ ID NO: 39,
(b) un acide nucléique qui est dégénéré par rapport à l'acide nucléique selon (a), et
(c) un acide nucléique qui présente, par rapport à l'acide nucléique sous (a) ou (b), une identité de séquences d'au moins 90%.
